# EUROPEAN PATENT SPECIFICATION

(11) **EP 4 020 489 B1**
(45) Date of publication and mention of the grant of the patent: **27.08.2025**
(21) Application number: 20919607.0
(22) Date of filing: 30.12.2020
(51) Int. Cl.: G16H 10/60, G16H 15/00, G16H 40/67, G16H 50/20

(54) **MULTI-PARAMETER MONITORING DEVICE DATA ANALYSIS REPORT GENERATION METHOD AND SYSTEM**
VERFAHREN UND SYSTEM ZUR GENERIERUNG VON DATENANALYSEBERICHTEN MIT MEHREREN PARAMETERN
PROCÉDÉ ET SYSTÈME DE GÉNÉRATION DE RAPPORT D'ANALYSE DE DONNÉES DE DISPOSITIF DE SURVEILLANCE À PARAMÈTRES MULTIPLES

(30) Priority: 18.02.2020 CN 202010100416
(43) Date of publication of application: 29.06.2022
(73) Proprietor: Shanghai SVM Medical Technology Co., Ltd., Shanghai 200233 (CN)
(72) Inventor: LONG, Yun, Shanghai 200233 (CN); HUANG, Xiaobo, Shanghai 200233 (CN); CHEN, Yundai, Shanghai 200233 (CN); SU, Longxiang, Shanghai 200233 (CN); LAN, Yunping, Shanghai 200233 (CN); ZHANG, Song, Shanghai 200233 (CN); ZHAI, Qian, Shanghai 200233 (CN); HAN, Baoshi, Shanghai 200233 (CN); HE, Hongli, Shanghai 200233 (CN); CAO, Haiquan, Shanghai 200233 (CN); WANG, Xinkang, Shanghai 200233 (CN); ZHENG, Xiangde, Shanghai 200233 (CN); WU, Yi, Shanghai 200233 (CN); SUN, Sinan, Shanghai 200233 (CN); WANG, Dong, Shanghai 200233 (CN); TANG, Zujun, Shanghai 200233 (CN); ZHU, Bin, Shanghai 200233 (CN); YANG, Haoyu, Shanghai 200233 (CN); SUN, Jian, Shanghai 200233 (CN); ZHANG, Jinjing, Shanghai 200233 (CN)
(74) Representative: Botti & Ferrari S.p.A.
(86) International application number: PCT/CN2020/141363
(87) International publication number: WO 2021/164436

(56) References cited:
- WO-A1-2014/005106
- WO-A1-2018/134432
- WO-A2-2004/075009
- CN-A- 101 363 842
- CN-A- 108 417 258
- CN-A- 108 447 529
- CN-A- 110 019 042
- CN-A- 111 128 331
- JP-A- 2007 094 943
- US-A1- 2018 018 428

## Description

### TECHNICAL FIELD

The present disclosure relates to the field of medical device data processing technologies, and in particular, to a method and system for generating a data analysis report of a multi-parameter monitoring device.

### BACKGROUND

As an important device in the medical field, a multi-parameter monitoring device is used to monitor changes in physiological and pathological data of critically ill patients. According to data change trends and evolution processes provided by the device, a doctor evaluates changes and severity of a patient's condition, so as to make a clinical decision and formulate a medical treatment plan. For example, a patient's heart rate and respiratory rate are on an upward trend, and a body temperature is on a downward/upward trend, based on which a doctor can determine that the patient's body is fighting an infection, thereby providing further diagnosis and treatment to prevent disease progression.

Over the past decade, the global multi-parameter monitoring device technology has made great progress, which possesses the ability to generate hundreds of MBytes of physiological and pathological data within 24 hours. However, clinical application modes of the multi-parameter data have not been advanced accordingly. The existing multi-parameter monitoring devices from various factories lack the technology to generate data analysis reports. Thus, when an alarm is issued, it is required that a medical staff retrieves data from a bedside multi-parameter monitoring device or a multi-parameter monitoring central workstation, and performs manual analysis on a device screen. In addition, it takes a lot of time to view all of the patient's data. In most cases, the medical staff only retrieves segment data for viewing. Recently, multi-parameter monitoring devices from some factories are equipped with a printing function, but it still takes a lot of time for the medical staff to view and select data on the screen, and the printout is only a record of partial data. Obviously, these two application modes of the multi-parameter data do not allow the medical staff to comprehensively understand change trends and processes of the patient's physiological and pathological data, thus being not conducive to diagnosis and treatment. Moreover, since the medical staff is generally not skilled at multi-parameter data analysis, there may be a risk of medical errors caused by missed diagnosis and misdiagnosis. At present, about tens of millions of multi-parameter monitoring devices are in use in hospitals around the world, and thus there is an urgent need to solve the above problem.

The patent publication No. WO2018134432A1 discloses a method for providing a report about an individual comprising the steps of: (i) providing a report generation system comprising a processor; (ii) receiving, by the processor, a command to generate a report comprising demographic information, medical/health information, and/or contextual information, where the contextual information comprises information, other than medical information, impacting the health or care of the individual; (iii) collecting, by the processor, medical information about the individual; (iv) collecting, by the processor, contextual information; (v) generating, by the processor, a report with the demographic information, medical/health information, and/or the contextual information; and (vi) communicating (160) the generated report to the individual or an intended recipient.

The patent publication No. CN110019042A discloses a report file generation method, device and system. The method includes: acquiring the report generation request of the client, wherein the report comprises the target data set and the report style corresponding to the target data set; obtaining data in the target data set, generating a corresponding report according to the report style corresponding to the target data set, and storing the generated report as a report file in a preset file format; sending the thumbnail of the report file to a client for a user to select.

### SUMMARY

In view of the above analysis, the present disclosure provides a method and system for generating a data analysis report of a multi-parameter monitoring device, aiming to solve the problem that the existing multi-parameter monitoring device technology lacks the ability to generate data analysis reports.

The objective of the present application is achieved by the following technical solutions.

An aspect provides a method for generating a data analysis report of a multi-parameter monitoring device, including:
receiving, by an application server, multi-parameter data transmitted in real time by the multi-parameter monitoring device, and storing the received multi-parameter data in a database server;
receiving, by the application server, a report generation instruction transmitted by a client, the instruction including a client identifier that is configured for indicating a permission of the client; and
in a case that the permission of the client is a first mode permission (active mode permission), transmitting, by the application server, the multi-parameter data to the client, to allow the client to process the multi-parameter data, so as to generate a multi-parameter analysis report and a report attachment; and in a case that the permission of the client is a second mode permission (passive mode permission), processing, by the application server, the multi-parameter data to generate the multi-parameter analysis report and the report attachment, and transmitting the multi-parameter analysis report and the report attachment to the client.

Further, after the application server receives the multi-parameter data transmitted in real time by the multi-parameter monitoring device, the method also includes: configuring a unique identifier for the multi-parameter data;
storing the configured unique identifier in a first storage area of the database server, and establishing a unique identifier index based on the unique identifier;
storing the received multi-parameter data in a second storage area of the database server, and marking the multi-parameter data according to the unique identifier of the multi-parameter data; and
storing the generated multi-parameter analysis report and the report attachment in a third storage area of the database server, and marking the generated multi-parameter analysis report and the report attachment according to the unique identifier of the multi-parameter data.

The unique identifier index established by the first storage area has a mutual mapping relationship with the second storage area and the third storage area, respectively, to provide unified management of information stored in the first storage area, the second storage area, and the third storage area that is associated by the unique identifier.

Further, after the application server receives the multi-parameter data transmitted in real time by the multi-parameter monitoring device, the method also includes:
counting, by the application server, a time length of the received multi-parameter data;
in a case that the time length of the received multi-parameter data reaches a predetermined time length, transmitting the unique identifier index of the multi-parameter data of the predetermined time length to the client;
in a case that the time length of the multi-parameter data is less than the predetermined time length and no longer increases, transmitting the unique identifier index of the multi-parameter data whose time length is less than the predetermined time length and no longer increases to the client; and
transmitting, by the client, the report generation instruction to the application server according to the received unique identifier index.

Further, the report generation instruction also includes a data time period parameter for report generation;
the application server determines to-be-processed multi-parameter data of a corresponding time period length based on the data time period parameter for report generation, and generate the multi-parameter analysis report and the report attachment based on the determined multi-parameter data of the corresponding time period length.

On the basis of the foregoing solution, following improvements have been made.

Further, the multi-parameter data includes patient information, waveform data, and numerical data.

Further, the generation of the multi-parameter analysis report and the report attachment includes:
processing the multi-parameter data, to obtain measurement statistical values, analysis statistical charts, time series identifiers, and event identifiers, the measurement statistical values including an average value, a maximum value, a minimum value, and a variability analysis value;
importing the measurement statistical values, the time series identifiers, the event identifiers, and patient basic information obtained by mapping the unique identifier index into a multi-parameter analysis report framework, to generate the multi-parameter analysis report; and
importing the measurement statistical values, the analysis statistical charts, the time series identifiers, the event identifiers, the multi-parameter waveform data, and the numerical data into a multi-parameter analysis report attachment framework, to generate the multi-parameter analysis report attachment.

The multi-parameter analysis report is configured for displaying a change trend of each parameter and a data analysis conclusion, and the report attachment is configured for displaying an analysis report of each parameter including the waveform data and the numerical data.

Further, the multi-parameter analysis report framework includes at least a patient basic information area; and
the multi-parameter analysis report framework also includes:
one or more of following parameter data trend analysis areas: a heart rate data trend analysis area, a blood pressure data trend analysis area, a respiratory rate data trend analysis area, a blood oxygen saturation level data trend analysis area, a body temperature data trend analysis area, and a designated data trend analysis area; and
a data analysis conclusion area.

Further, the report generation instruction also includes a setting parameter of the designated data trend analysis area, according to which the application server sets content of the designated data trend analysis area; and
the report generation instruction also includes a setting parameter of the data analysis conclusion area, according to which the application server sets content of the data analysis conclusion area.

Further, the parameter data trend analysis area has an ordinate set as a numerical scale and an abscissa set as a time scale, configured for displaying a state in a unit time and a change process of each parameter, and includes at least the measurement statistical values, the time series identifiers, and the event identifiers of each parameter.

Further, the designated data trend analysis area is configured for displaying a trend analysis graph of parameter data that is preset and needs intensive monitoring for patients with different diseases.

The parameter data that needs intensive monitoring includes one or more of following parameters: a perfusion index, a partial pressure of end-tidal carbon dioxide, an airway flow/pressure, a central venous pressure, a pulse pressure, a stroke volume, an intracranial pressure, and a bispectral index.

Further, each of the parameter data trend analysis areas in the multi-parameter analysis report is associated with each parameter analysis page in the report attachment. In response to clicking on a certain data trend analysis area on a screen displaying the multi-parameter analysis report, the multi-parameter analysis report automatically jumps to a corresponding parameter analysis page, to allow a user to view page by page the measurement statistical values, the analysis statistical charts, the waveform data, the numerical data, and marked event data, and perform a zoom in operation, a zoom out operation, or a scrolling operation on the screen for viewing, and select a range of data for viewing.

Further, the client with the first mode permission and the client with the second mode permission are also configured to transmit the multi-parameter analysis report, the report attachment, and the unique identifier index to an associated medical staff terminal, after the multi-parameter analysis report and the report attachment are generated.

Further, the method also includes:
receiving, by the application server, a report acquisition instruction transmitted by the medical staff terminal, the report acquisition instruction including a unique identifier index of a patient or a statistical parameter;
in a case that the unique identifier index is included, retrieving, by the application server, a multi-parameter analysis report and a report attachment according to the unique identifier index, and transmitting the multi-parameter analysis report and the report attachment to the medical staff terminal; and
in a case that the statistical parameter is included, retrieving, by the application server, a multi-parameter analysis report and a report attachment that satisfy the statistical parameter, and transmitting the multi-parameter analysis report and the report attachment to the medical staff terminal.

Further, the method also includes:
receiving, by the application server, a report archiving instruction transmitted by a client or a medical staff terminal with a report archiving permission, the report archiving instruction including the unique identifier index; and retrieving, by the application server, a multi-parameter analysis report and a report attachment according to the unique identifier index, and transmitting the multi-parameter analysis report and the report attachment to a hospital information management system, an electronic medical record system, or a critical clinical information system, to store the multi-parameter analysis report and the report attachment in a patient electronic medical record.

Further, the application server receiving the report generation instruction transmitted by the client also includes:
receiving, by the application server, the report generation instruction that is transmitted by a client browser and successfully authenticated by a world wide web (WEB) server, the report generation instruction including a client browser identifier, and the permission of the client indicated by the client browser identifier being the second mode permission.

Further, the application server receiving the multi-parameter data transmitted in real time by the multi-parameter monitoring device includes:
connecting, by a mobile storage apparatus, to an interface of the multi-parameter monitoring device, to download the multi-parameter data; and
connecting, by the client, to an interface of the mobile storage apparatus, to upload read multi-parameter data to the application server.

Another aspect provides a system for generating a data analysis report of a multi-parameter monitoring device, including:
one or more clients, configured to transmit a report generation instruction to an application server, the report generation instruction including a client identifier that is configured for indicating a permission of the client;
the application server, configured to receive multi-parameter data transmitted in real time by the multi-parameter monitoring device; and further configured to receive the report generation instruction transmitted by the client, in a case that the permission of the client is a first mode permission, transmit the multi-parameter data to the client, to allow the client to process the multi-parameter data, so as to generate a multi-parameter analysis report and a report attachment, and in a case that the permission of the client is a second mode permission, process the multi-parameter data to generate the multi-parameter analysis report and the report attachment, and transmit the multi-parameter analysis report and the report attachment to the client; and
a database server, configured to store the multi-parameter data received by the application server.

The application server is also configured to configure a unique identifier for the multi-parameter data after receiving the multi-parameter data transmitted in real time by the multi-parameter monitoring device.

The database server includes a first storage area, a second storage area, and a third storage area.

The first storage area of the database server is configured to store the configured unique identifier, and establish a unique identifier index based on the unique identifier.

The second storage area of the database server is configured to store the multi-parameter data received by the application server, and mark the multi-parameter data according to the unique identifier of the multi-parameter data.

The third storage area of the database server is configured to store the generated multi-parameter analysis report and the report attachment, and mark the generated multi-parameter analysis report and the report attachment according to the unique identifier of the multi-parameter data.

The unique identifier index established by the first storage area has a mutual mapping relationship with the second storage area and the third storage area, respectively, to provide unified management of information stored in the first storage area, the second storage area, and the third storage area that is associated by the unique identifier.

Further, after receiving the multi-parameter data transmitted in real time by the multi-parameter monitoring device, the application server is configured to perform the following operations:
counting a time length of the received multi-parameter data;
in a case that the time length of the received multi-parameter data reaches a predetermined time length, transmitting the unique identifier index of the multi-parameter data of the predetermined time length to the client; and
in a case that the time length of the multi-parameter data is less than the predetermined time length and no longer increases, transmitting the unique identifier index of the multi-parameter data whose time length is less than the predetermined time length and no longer increases to the client.

The client is also configured to transmit the report generation instruction to the application server according to the received unique identifier index.

Further, the report generation instruction also includes a data time period parameter for report generation.

The application server is also configured to determine to-be-processed multi-parameter data of a corresponding time period length based on the data time period parameter for report generation, and generate the multi-parameter analysis report and the report attachment based on the determined multi-parameter data of the corresponding time period length.

On the basis of the foregoing solution, following improvements have been made.

Further, the multi-parameter data includes patient information, waveform data, and numerical data.

Further, the generation of the multi-parameter analysis report and the report attachment includes:
processing the multi-parameter data, to obtain measurement statistical values, analysis statistical charts, time series identifiers, and event identifiers, the measurement statistical values including an average value, a maximum value, a minimum value, and a variability analysis value;
importing the measurement statistical values, the time series identifiers, the event identifiers, and patient basic information obtained by mapping the unique identifier index into a multi-parameter analysis report framework, to generate the multi-parameter analysis report; and
importing the measurement statistical values, the analysis statistical charts, the time series identifiers, the event identifiers, the multi-parameter waveform data, and the numerical data into a multi-parameter analysis report attachment framework, to generate the multi-parameter analysis report attachment.

The multi-parameter analysis report is configured for displaying a change trend of each parameter and a data analysis conclusion, and the report attachment is configured for displaying an analysis report of each parameter including the waveform data and the numerical data.

Further, the multi-parameter analysis report framework includes at least a patient basic information area; and
the multi-parameter analysis report framework further includes:
one or more of following parameter data trend analysis areas: a heart rate data trend analysis area, a blood pressure data trend analysis area, a respiratory rate data trend analysis area, a blood oxygen saturation level data trend analysis area, a body temperature data trend analysis area, and a designated data trend analysis area; and
a data analysis conclusion area.

Further, the report generation instruction also includes a setting parameter of the designated data trend analysis area, according to which the application server sets content of the designated data trend analysis area; and
the report generation instruction also includes a setting parameter of the data analysis conclusion area, according to which the application server sets content of the data analysis conclusion area.

Further, the parameter data trend analysis area has an ordinate set as a numerical scale and an abscissa set as a time scale, configured for displaying a state in a unit time and a change process of each parameter, and includes at least the measurement statistical values, the time series identifiers, and the event identifiers of each parameter.

Further, the designated data trend analysis area is configured for displaying a trend analysis graph of parameter data that is preset and needs intensive monitoring for patients with different diseases.

The parameter data that needs intensive monitoring includes one or more of following parameters: a perfusion index, a partial pressure of end-tidal carbon dioxide, an airway flow/pressure, a central venous pressure, a pulse pressure, a stroke volume, an intracranial pressure, and a bispectral index.

Further, each of the parameter data trend analysis areas in the multi-parameter analysis report is associated with each parameter analysis page in the report attachment. In response to clicking on a certain data trend analysis area on a screen displaying the multi-parameter analysis report, the multi-parameter analysis report automatically jumps to a corresponding parameter analysis page, to allow a user to view page by page the measurement statistical values, the analysis statistical charts, the waveform data, the numerical data, and marked event data, and perform a zoom in operation, a zoom out operation, or a scrolling operation on the screen for viewing, and select a range of data for viewing.

Further, the client with the first mode permission and the client with the second mode permission are also configured to transmit the multi-parameter analysis report, the report attachment, and the unique identifier index to an associated medical staff terminal, after the multi-parameter analysis report and the report attachment are generated.

Further, the system also includes a medical staff terminal, configured to transmit a report acquisition instruction to the application server, the report acquisition instruction including a unique identifier index of a patient or a statistical parameter.

The application server is also configured to receive the report acquisition instruction transmitted by the medical staff terminal, in a case that the unique identifier index is included, retrieve a multi-parameter analysis report and a report attachment according to the unique identifier index, and transmit the multi-parameter analysis report and the report attachment to the medical staff terminal; and
in a case that the statistical parameter is included, retrieve a multi-parameter analysis report and a report attachment that satisfy the statistical parameter, and transmit the multi-parameter analysis report and the report attachment to the medical staff terminal.

Further, the application server is also configured to receive a report archiving instruction transmitted by a client or a medical staff terminal with a report archiving permission, the report archiving instruction including the unique identifier index, retrieve a multi-parameter analysis report and a report attachment according to the unique identifier index, and transmit the multi-parameter analysis report and the report attachment to a hospital information management system, an electronic medical record system, or a critical clinical information system, to store the multi-parameter analysis report and the report attachment in a patient electronic medical record.

Further, the system also includes a WEB server.

The application server is configured to receive the report generation instruction that is transmitted by a client browser and successfully authenticated by the WEB server, the report generation instruction including a client browser identifier, and the permission of the client indicated by the client browser identifier being the second mode permission.

Further, the system also includes a mobile storage apparatus.

The application server is configured to receive the multi-parameter data transmitted by the multi-parameter monitoring device, including:
the mobile storage apparatus connects to an interface of the multi-parameter monitoring device, to download the multi-parameter data; and
the client connects to an interface of the mobile storage apparatus, to upload read multi-parameter data to the application server.

The present disclosure has the following beneficial effects.

According to the method and system for generating a data analysis report of a multi-parameter monitoring device according to the present disclosure:
1. The method and system can make full use of the multi-parameter data outputted by the multi-parameter monitoring device, to calculate to generate the multi-parameter analysis report and the report attachment. By viewing the multi-parameter analysis report and the report attachment, the medical staff can quickly and comprehensively understand change trends and processes of patient's physiological and pathological data, so as to formulate a diagnosis and treatment plan and a nursing care plan, which improves quality and efficiency of medical treatment. The present disclosure overcomes the deficiency that the existing technology lacks the ability to generate the multi-parameter analysis report, thus having a wide range of applicability.
2. The present disclosure has good distribution and scalability, which is able to manage large-scale multi-parameter monitoring devices, client devices, and medical staff terminals. A hospital can centrally process multi-parameter data services with a huge amount of data scattered in various departments, and permanently save the multi-parameter data and the report files, which improves work efficiency and reduces service costs. The present disclosure may also be applied to provide data services for multi-parameter monitoring devices in lower-level hospitals, which overcomes the shortcomings of the existing technology, thus having a wide range of applicability.
3. The multi-parameter analysis report and the report attachment generated according to the present disclosure provide the change trends, the event statuses and the time points of each parameter data during a patient's medical treatment process, as well as the data analysis conclusion, so that the medical staff can understand the change trends and processes of the patient's physiological and pathological data and the disease severity, so as to provide a precise intervention, which improves quality and efficiency of medical treatment, thereby alleviating work burden on the medical staff. The present disclosure overcomes the deficiencies of the existing technology in the clinical application, thus having a wide range of applicability.

The above technical solutions in the present disclosure can also be combined with each other to realize more preferred combination solutions thereof. Other features and advantages of the present application will be described in the following specification, and some of these will become apparent from the specification or be understood by implementing the present application. The objectives and other advantages of the present application can be implemented or obtained by structures specifically indicated in the written specification, claims, and accompanying drawings.

### BRIEF DESCRIPTION OF THE DRAWINGS

The accompanying drawings are provided merely for illustrating specific examples and are not considered as limiting the present application. Throughout the accompanying drawings, the same reference numerals represent the same components.
FIG. 1 is a flowchart of a method for generating a data analysis report of a multi-parameter monitoring device according to embodiment 1 of the present disclosure.
FIG. 2 is a framework diagram of a multi-parameter data analysis report generated according to embodiment 1 of the present disclosure.
FIG. 3 is a schematic diagram of viewing an attachment of a multi-parameter analysis report generated according to embodiment 1 of the present disclosure.
FIG. 4 is a framework diagram of a system for generating a data analysis report of a multi-parameter monitoring device in a client/server (C/S) architecture according to embodiment 3, which does not describe part of the claimed invention.
FIG. 5 is a framework diagram of a system for generating a data analysis report of a multi-parameter monitoring device in a browser/server (B/S) architecture according to embodiment 3, which does not describe part of the claimed invention.
FIG. 6 is a framework diagram of a system for generating a data analysis report of a multi-parameter monitoring device in a local client architecture according to embodiment 3, which does not describe part of the claimed invention.

### DETAILED DESCRIPTION OF THE EMBODIMENTS

Preferred examples of the present application will be described in detail below with reference to the accompanying drawings. The accompanying drawings constitute a part of the present application, and are used together with the examples of the present application for explaining principles of the present application rather than for limiting a scope of the present application.

### Embodiment 1

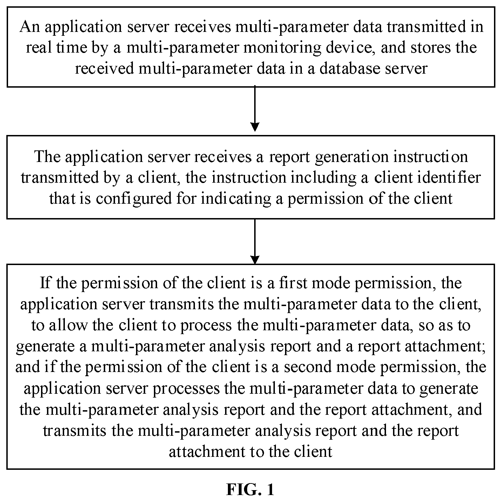

A specific embodiment of the present disclosure provides a method for generating a data analysis report of a multi-parameter monitoring device, a flowchart of which is shown in FIG. 1. The method includes the following operations:
Step S1. An application server receives multi-parameter data transmitted in real time by the multi-parameter monitoring device, and stores the received multi-parameter data in a database server.
Step S2. The application server receives a report generation instruction transmitted by a client. The instruction includes a client identifier, which is configured for indicating a permission of the client.
Step S3. If the permission of the client is a first mode permission, the application server transmits the multi-parameter data to the client, to allow the client to process the multi-parameter data, so as to generate a multi-parameter analysis report and a report attachment. If the permission of the client is a second mode permission, the application server processes the multi-parameter data to generate the multi-parameter analysis report and the report attachment, and transmits the multi-parameter analysis report and the report attachment to the client.

After the multi-parameter analysis report and the report attachment are generated, the method may also include the following operation:
Step S4. After the multi-parameter analysis report and the report attachment are generated, the client with the first mode permission and the client with the second mode permission transmit the multi-parameter analysis report and the report attachment to an associated medical staff terminal.

Compared with the related art, the method in the present disclosure can make full use of the multi-parameter data outputted by the multi-parameter monitoring device, to generate the multi-parameter analysis report and the report attachment. By viewing the multi-parameter analysis report and the report attachment, the medical staff can quickly and comprehensively understand change trends and processes of patient's physiological and pathological data, so as to formulate a diagnosis and treatment plan and a nursing care plan. In addition, the present disclosure has good distribution and scalability, which has the ability to manage large-scale multi-parameter monitoring devices, client devices, and medical staff terminals. The hospital can centrally process multi-parameter data services with a huge amount of data scattered in various departments, and permanently save the multi-parameter data and the report files, thereby reducing service costs. The medical staff terminal may view, retrieve, and query report files at any time, which improves quality and efficiency of medical treatment. Moreover, the present disclosure may receive data from a multi-parameter device at a low-level hospital and then provide the multi-parameter analysis report and the report attachment to the low-level hospital, which overcomes the deficiencies of the existing technology in the clinical application, thus having a wide range of applicability.

The method provided by the present disclosure is adapted for an actual situation of a medical process. Specifically, in the medical field, since the data analysis is highly professional and the medical staff is generally not skilled at the data analysis, there may exist a risk of medical errors caused by missed diagnosis and misdiagnosis. Thus, the client is provided in the method, configured to cooperate with the application server for generating the multi-parameter analysis report and the report attachment.

Optionally, in the method, the permission of the client may be determined according to different application scenarios and requirements of hospitals, which is not limited herein. For example, in some embodiments, the hospital may only limit the functions of the application server.

In this embodiment, the permission of the client refers to whether the client itself has a report generation function, which may be indicated by a client device identifier. For the purpose of system management and system security, all client device identifiers indicating the report generation function need to be stored in the application server. In this case, the client with the first mode permission can generate the multi-parameter analysis report and the report attachment relying on its excellent storage capacity and computing power; that is, the client with the first mode permission itself has the report generation function. For example, the client device identifier may be a desktop computer. The client with the second mode permission does not have the report generation function due to its weak storage capacity and computing power, and in this case, the application server needs to be used to generate the multi-parameter analysis report and the report attachment. For example, the client identifier may be a mobile device. Accordingly, Step 3 includes the following operations:
Step S31. The application server determines whether the client device identifier has been stored. If the client device identifier has been stored, step S32 is performed; and if the client device identifier has not been stored, the application server transmits feedback information of "not having the report generation function" to the client.
Step S32. If the client device identifier is the desktop computer, the application server transmits the multi-parameter data to the client, to allow the client to process the multi-parameter data, so as to generate the multi-parameter analysis report and the report attachment. If the client device identifier is the mobile device, the application server processes the multi-parameter data to generate the multi-parameter analysis report and the report attachment.

It can be envisaged that as technologies advance, the mobile device will be developed to have more powerful storage capacity and computing power. At that time, the application server may redefine the client device identifier, to determine the process of generating the multi-parameter analysis report and the report attachment is carried out in the client or in the application server.

In other embodiments, the hospital may limit the functions of the client and the application server at the same time. The permission of the client may be specifically set according to an actual situation, which is more flexible. It is necessary to limit the application server correspondingly, to ensure that the application server effectively identifies the permission of the client, and determines the process of generating the multi-parameter analysis report and the report attachment is carried out in the client or in the application server according to the permission of the client.

In the above step S1, after the application server receives the multi-parameter data transmitted in real time by the multi-parameter monitoring device, the method also includes: configuring a unique identifier for the multi-parameter data; storing the configured unique identifier in a first storage area of the database server, and establishing a unique identifier index based on the unique identifier. For example, the unique identifier may be a combination of various pieces of clinical information and monitoring information, such as a device number, a hospitalization number, a data sampling time, and etc. The unique identifier index is a storage structure that points to data and is established in the first storage area of the database server based on the unique identifier, which is configured for data retrieval, query, statistical, and management. The application server also stores the received multi-parameter data in a second storage area of the database server, and marks the multi-parameter data according to the unique identifier of the multi-parameter data. In addition, the application server also stores the generated multi-parameter analysis report and report attachment in a third storage area of the database server, and marks the generated multi-parameter analysis report and report attachment according to the unique identifier of the multi-parameter data. The unique identifier index established by the first storage area has a mutual mapping relationship with the second storage area and the third storage area, respectively, to realize unified management of information stored in the first storage area, the second storage area, and the third storage area that is associated by the unique identifier. In this way, the unified management of the multi-parameter data and the report files can be realized, which facilitates retrieval of the multi-parameter data and the report files, data service scheduling, and statistical analysis.

In the above step S1, after the application server receives the multi-parameter data transmitted in real time by the multi-parameter monitoring device, the method also includes: the application server counting a time length of the received multi-parameter data; if the time length of the received multi-parameter data reaches a predetermined time length, transmitting the unique identifier index of the multi-parameter data of the predetermined time length to the client; and if the time length of the multi-parameter data is less than the predetermined time length and no longer increases, transmitting the unique identifier index of the multi-parameter data whose time length is less than the predetermined time length and no longer increases to the client. The client transmits the report generation instruction to the application server according to the received unique identifier index. In this way, the multi-parameter analysis report and the report attachment can be generated regularly, thereby better satisfying user requirements. The predetermined time length may be set according to user requirements or patient diagnosis and treatment requirements, such as, 24 hours.

In an actual application, there is a requirement to temporarily generate an analysis report of a specific time period. In this case, a staff sets a data time period parameter by the client, and load the data time period parameter in the report generation instruction that is to be transmitted to the application server. Based on the data time period parameter for report generation, the application server determines to-be-processed multi-parameter data of a corresponding time period length, and generates the multi-parameter analysis report and the report attachment according to the determined multi-parameter data of the corresponding time period length.

Optionally, in the above step S1, the application server may receive the multi-parameter data transmitted in real time by the multi-parameter monitoring device in a variety of manners, such as, in a wireless/wired network transmission manner. In a case that network transmission is not allowed in certain environments, the multi-parameter data may be transmitted as follows to ensure a smooth process: a mobile storage apparatus is connected to an interface of the multi-parameter monitoring device, to download the multi-parameter data; and the client is connected to an interface of the mobile storage apparatus, to upload the read multi-parameter data to the application server.

Optionally, the multi-parameter monitoring device includes at least a multi-parameter monitor and a multi-parameter monitoring central workstation. The multi-parameter data includes patient information, waveform data, and numerical data. The client and the application server both perform the following process to generate the multi-parameter analysis report and the report attachment.

The multi-parameter data is processed, to obtain measurement statistical values, time series identifiers, event identifiers, and analysis statistical charts. The analysis statistical charts may be selected from one or more of the following: a bar graph, a pie chart, a line graph, a histogram, a scatter plot, and a variability analysis chart. The measurement statistical values include: an average value, a maximum value, a minimum value, and a variability analysis value. The measurement statistical values, the time series identifiers, the event identifiers, and patient basic information obtained by mapping the unique identifier index are imported into a multi-parameter analysis report framework, to generate the multi-parameter analysis report. The measurement statistical values, the analysis statistical charts, the time series identifiers, the event identifiers, the multi-parameter waveform data, and the numerical data are imported into a multi-parameter analysis report attachment framework, to generate the multi-parameter analysis report attachment.

The multi-parameter analysis report is configured to display a change trend of each parameter and a data analysis conclusion. The report attachment is configured to display an analysis report of each parameter including the multi-parameter waveform data and the numerical data.

Optionally, the multi-parameter analysis report framework includes at least a patient basic information area; and in addition, includes one or more of the following parameter data trend analysis areas: a heart rate data trend analysis area, a blood pressure data trend analysis area, a respiratory rate data trend analysis area, a blood oxygen saturation level data trend analysis area, a body temperature data trend analysis area, and a designated data trend analysis area; and a data analysis conclusion area.

Optionally, when the multi-parameter analysis report framework includes the designated data trend analysis area, the report generation instruction also includes a setting parameter of the designated data trend analysis area, according to which the application server sets content of the designated data trend analysis area. It should be noted that the designated data trend analysis area is configured to display a trend analysis graph of parameter data that is preset and needs intensive monitoring for patients with different diseases. The parameter data needing the intensive monitoring includes one or more of the following: a perfusion index, a partial pressure of end-tidal carbon dioxide, an airway flow/pressure, a central venous pressure, a pulse pressure, a stroke volume, an intracranial pressure, and a bispectral index. In addition, the report generation instruction also includes a setting parameter of the data analysis conclusion area, according to which the application server sets content of the data analysis conclusion area.

Optionally, the parameter data trend analysis area has an ordinate set as a numerical scale and an abscissa set as a time scale, configured for displaying a state in a unit time and a change process of each parameter; and in addition, includes at least the measurement statistical values, the time series identifiers, and the event identifiers of each parameter. Each parameter data trend analysis area in the multi-parameter analysis report is associated with each parameter analysis page in the report attachment. In response to clicking on a certain data trend analysis area on a screen displaying the multi-parameter analysis report, the multi-parameter analysis report automatically jumps to the corresponding parameter analysis page. In this way, the medical staff can view page by page the measurement statistical values, the analysis statistical charts, the waveform data, the numerical data and the marked event data, and perform a zoom in operation, a zoom out operation, or a scrolling operation on the screen, and select a range of data for viewing.

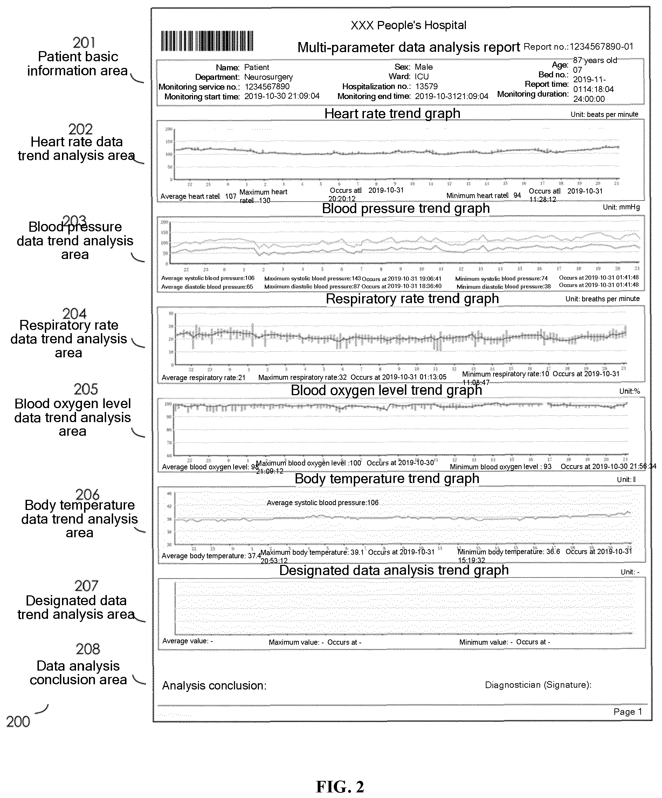

A framework of a multi-parameter analysis report 200 as shown in FIG. 2 includes a patient basic information area 201, a heart rate data trend analysis area 202, a blood pressure data trend analysis area 203, a respiratory rate data trend analysis area 204, and a blood oxygen saturation level data trend analysis area 205, a body temperature data trend analysis area 206, a designated data trend analysis area 207, and a data analysis conclusion area 208. The system software or the data analysis report generation software imports the measurement statistical values, the time series identifiers, and the event identifiers of each parameter into the multi-parameter analysis report framework, to generate the multi-parameter analysis report 200.

The patient basic information area 201 in this embodiment includes at least a name, an age, a sex, an ethnicity, a department and ward, a bed number, a hospitalization number, a disease type, and monitoring start and end time of a patient.

The heart rate data trend analysis area 202, the blood pressure data trend analysis area 203, the respiratory rate data trend analysis area 204, the blood oxygen saturation level data trend analysis area 205, the body temperature data trend analysis area 206, and the designated data trend analysis area 207 in this embodiment have an ordinate set as a numerical scale and an abscissa set as a time scale, configured for displaying a state in a unit time and a change process of each parameter; and in addition, includes at least the measurement statistical values, the time series identifiers and the event identifiers of each parameter. The heart rate data trend analysis area 202 in FIG. 2 is used as an example. The multi-parameter data to be processed is divided into several data segments of unit time. The heart rate measurement statistical values (including the average value, the maximum value, and the minimum value) of each data segment of unit time and the multi-parameter data to be processed are calculated. Then, the heart rate trend graph is drawn using the heart rate average values of the data segments in the form of the line graph, and the measurement statistical values are listed under the trend graph.

The designated data trend analysis area 207 in this embodiment is configured for displaying a trend analysis of certain parameter data that is designated by a doctor and needs intensive monitoring for patients with different diseases (such as patients with respiratory failure, and patients in shock), which includes at least a perfusion index, an end-tidal carbon dioxide, an airway flow/pressure, a central venous pressure, a pulse pressure, a stroke volume, an intracranial pressure, and a bispectral index.

The data analysis conclusion area 208 in this embodiment is configured for displaying a preliminary conclusion obtained through comprehensive analysis of the changes of various parameters, and a signature of an analysis technician or a doctor in charge of the data service.

The multi-parameter analysis report 200 in this embodiment is also provided with an attachment frame constituted by parameter analysis pages. The parameter analysis pages use analysis report templates that are commonly used in relevant medical disciplines, such as analysis report templates of electrocardiography, blood pressure, respiration, blood oxygen saturation level, body temperature, central venous pressure, invasive blood pressure, intracranial pressure, and electroencephalography (For example, the parameter analysis page of electrocardiography data uses a commonly used dynamic electrocardiography analysis report template, and the parameter analysis page of non-invasive blood pressure uses a commonly used dynamic blood pressure analysis report template). The system software or the data analysis report generation software imports the measurement statistical values, the analysis statistical charts, the waveform data, the numerical data, the time series identifiers, and the event identifiers into the analysis page templates, so as to generate the attachment of the multi-parameter analysis report. These analysis report templates are commonly used, thus are not detailed herein.

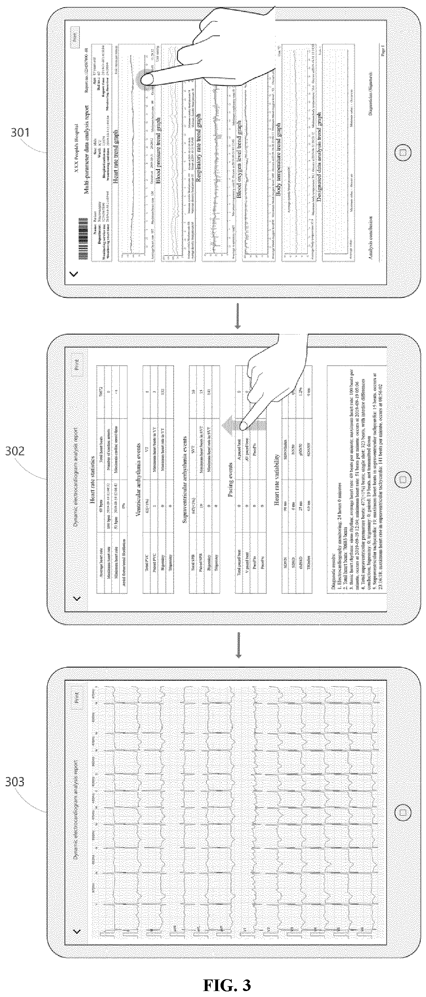

FIG. 3 shows a schematic diagram of viewing an attachment of the multi-parameter analysis report. Under the control of the medical staff terminal device, each parameter trend analysis area in the multi-parameter analysis report 200 is associated with each parameter analysis page in the attachment. As shown in FIG. 301, in response to a medical staff clicking on the heart rate trend analysis area on the screen, the current page automatically jumps to a dynamic electrocardiogram report page as shown in FIG. 302, so that the medical staff can view the measurement statistical values and abnormal events statistical values of the patent's electrocardiography data obtained during the multi-parameter monitoring process. By sliding on the screen of the terminal device, the medical staff can view page by page the statistical charts, the heart rate variability analysis chart, the waveform data as shown in FIG. 303, the numerical data, and marked event data. In addition, the medical staff can perform a zoom in operation, a zoom out operation, or a scrolling operation on the screen for viewing, and select a range of data for viewing.In response to clicking a return button on the screen by the medical staff, the page being currently displayed on the screen may automatically jump to the multi-parameter analysis report 200. This allows the medical staff to quickly understand the change processes of the patient's physiological and pathological data, and related data details, which improves medical quality and work efficiency, thereby reducing the work burden on medical staffs.

Compared with the related art, the multi-parameter analysis report 200 in this embodiment and further solutions show the change process, the event statuses and the time points of each parameter data that the medical staff pays close attention to, as well as the data analysis conclusion. Based on the data changes, the medical staff can formulate the diagnosis and treatment plan and the nursing care plan. Also, the medical staff can access patient's history reports for comparison and analysis, which exploits the value of the multi-parameter data in diagnosis and treatment. The attachment of the multi-parameter analysis report may be viewed on the medical staff terminal device, which helps the medical staff to understand more details about each parameter data. This improves medical quality and work efficiency, thereby overcoming the deficiencies of the existing multi-parameter monitoring device technology.

This embodiment provides the change trends and processes of the patient's physiological and pathological data during the multi-parameter monitoring process, which comprehensively reflects patient's medical condition and treatment effect. According to the change trend of each parameter, the medical staff can quickly determine disease progression and severity, so as to make an early detection of complications, and thereby providing further diagnosis and treatment to prevent deterioration. In addition, the medical staff gains more details about the data with the knowledge of the attachment. This embodiment improves medical quality and work efficiency, as well as reduces the work burden on the medical staff, thus having a wide range of applicability.

The multi-parameter analysis report and the report attachment generated in the present disclosure enable the medical staff to quickly and comprehensively obtain patient's physiological and pathological information, so as to provide a more targeted diagnosis and treatment plan and nursing care plan, which effectively alleviates the work burden on the medical staff. Therefore, after the multi-parameter analysis report and the report attachment are generated, the client with the first mode permission and the client with the second mode permission may also transmit the multi-parameter analysis report, the report attachment, and the unique identifier index to an associated medical staff terminal. The medical staff terminal is installed with the data management software, which is configured to perform data interaction with the client and the application server, and allow the medical staff to view the multi-parameter analysis report and the attachment. The medical staff terminal includes at least one or more of the following: a desktop computer, a large touch liquid crystal display screen, a mobile device.

Optionally, the application server also receives a report acquisition instruction transmitted by the medical staff terminal. The report acquisition instruction includes a unique identifier index of a patient or a statistical parameter. **In** a case that the unique identifier index is included, the application server retrieves a multi-parameter analysis report and a report attachment according to the unique identifier index, and transmits the multi-parameter analysis report and the report attachment to the medical staff terminal. In a case that the statistical parameter is included, the application server retrieves a multi-parameter analysis report and a report attachment that satisfy the statistical parameter, and transmits the multi-parameter analysis report and the report attachment to the medical staff terminal. Optionally, the application server further receives a report archiving instruction transmitted by the client or the medical staff terminal with a report archiving permission. The report archiving instruction includes the unique identifier index. The application server retrieves a multi-parameter analysis report and a report attachment according to the unique identifier index, and transmits the multi-parameter analysis report and the report attachment to a hospital information management system, an electronic medical record system, or a critical clinical information system, so as to store the multi-parameter analysis report and the report attachment in an electronic medical record of the patient.

The above solution solves the problem that the existing electronic medical records do not store the multi-parameter analysis report files. In addition, the report files in the text format effectively reduce the pressure of the huge amount of multi-parameter data on an upper-level information management system. In this embodiment, the client controls the application server to transmit the report files or a link to the medical staff terminal device for viewing. The medical staff terminal device may log in the system, to search and retrieve the data and the report files, which frees the medical staff from tedious work of manually inputting and modifying various vital sign data charts. The multi-parameter data forms a closed loop between clinical application and hospital management, which improves medical quality and work efficiency, as well as enhances the quality of the electronic medical records, thereby reducing the work burden on the medical staff. Thus, the present disclosure has a broad range of applicability.

### Embodiment 2

Another embodiment of the present disclosure provides a system for generating a data analysis report of a multi-parameter monitoring device, including: one or more clients, configured to transmit a report generation instruction to an application server, the report generation instruction including a client identifier that is configured for indicating a permission of the client; the application server, configured to receive multi-parameter data transmitted in real time by the multi-parameter monitoring device; and further configured to receive the report generation instruction transmitted by the client, in a case that the permission of the client is a first mode permission, transmit the multi-parameter data to the client, to allow the client to process the multi-parameter data, so as to generate a multi-parameter analysis report and a report attachment, and in a case that the permission of the client is a second mode permission, process the multi-parameter data to generate the multi-parameter analysis report and the report attachment, and transmit the multi-parameter analysis report and the report attachment to the client; and a database server, configured to store the multi-parameter data received by the application server.

Optionally, the system also includes a world wide web (WEB) server. The client transmits the report generation instruction to the application server through the WEB server.

Optionally, the system also includes a mobile storage apparatus. The application server receives the multi-parameter data transmitted by the multi-parameter monitoring device, including: the mobile storage apparatus connecting to an interface of the multi-parameter monitoring device, to download the multi-parameter data; and the client connecting to an interface of the mobile storage apparatus, to upload the read multi-parameter data to the application server.

The application server is also configured to configure a unique identifier for the multi-parameter data after receiving the multi-parameter data transmitted in real time by the multi-parameter monitoring device. The database server includes a first storage area, a second storage area, and a third storage area. The first storage area of the database server is configured to store the configured unique identifier, and establish a unique identifier index based on the unique identifier. The second storage area of the database server is configured to store the multi-parameter data received by the application server, and mark the multi-parameter data according to the unique identifier of the multi-parameter data. The third storage area of the database server is configured to store the generated multi-parameter analysis report and the report attachment, and mark the generated multi-parameter analysis report and the report attachment according to the unique identifier of the multi-parameter data. The unique identifier index established by the first storage area has a mutual mapping relationship with the second storage area and the third storage area, respectively, to realize unified management of information stored in the first storage area, the second storage area, and the third storage area that is associated by the unique identifier.

After receiving the multi-parameter data transmitted in real time by the multi-parameter monitoring device, the application server is also configured to perform the following operations: counting a time length of the received multi-parameter data; if the time length of the received multi-parameter data reaches a predetermined time length, transmitting the unique identifier index of the multi-parameter data of the predetermined time length to the client; and if the time length of the multi-parameter data is less than the predetermined time length and no longer increases, transmitting the unique identifier index of the multi-parameter data whose time length is less than the predetermined time length and no longer increases to the client. In this case, the client transmits the report generation instruction to the application server according to the received unique identifier index, so as to process the multi-parameter data to generate the multi-parameter analysis report and the report attachment.

The report generation instruction also includes a data time period parameter for report generation. The application server is also configured to determine to-be-processed multi-parameter data of a corresponding time period length based on the data time period parameter for report generation, and generate the multi-parameter analysis report and the report attachment based on the determined multi-parameter data of the corresponding time period length.

Optionally, the multi-parameter data includes patient information, waveform data, and numerical data.

Optionally, the generation of the multi-parameter analysis report and the report attachment includes: processing the multi-parameter data, to obtain measurement statistical values, analysis statistical charts, time series identifiers, and event identifiers, the measurement statistical values including an average value, a maximum value, a minimum value, and a variability analysis value; importing the measurement statistical values, the time series identifiers, the event identifiers, and patient basic information obtained by mapping the unique identifier index into a multi-parameter analysis report framework, to generate the multi-parameter analysis report; and importing the measurement statistical values, the analysis statistical charts, the time series identifiers, the event identifiers, the waveform data, and the numerical data into a multi-parameter analysis report attachment framework, to generate the report attachment. The multi-parameter analysis report is configured for displaying a change trend of each parameter and a data analysis conclusion, and the report attachment is configured for displaying an analysis report of each parameter including the waveform data and the numerical data.

Optionally, the multi-parameter analysis report framework includes at least a patient basic information area, and further includes one or more of following parameter data trend analysis areas: a heart rate data trend analysis area, a blood pressure data trend analysis area, a respiratory rate data trend analysis area, a blood oxygen saturation level data trend analysis area, a body temperature data trend analysis area, and a designated data trend analysis area; and a data analysis conclusion area.

Optionally, the report generation instruction also includes a setting parameter of the designated data trend analysis area, according to which the application server sets content of the designated data trend analysis area; and the report generation instruction also includes a setting parameter of the data analysis conclusion area, according to which the application server sets content of the data analysis conclusion area.

Optionally, the parameter data trend analysis area has an ordinate set as a numerical scale and an abscissa set as a time scale, configured for displaying a state in a unit time and a change process of each parameter, and includes at least the measurement statistical values, the time series identifiers, and the event identifiers of each parameter.

Optionally, the designated data trend analysis area is configured for displaying a trend analysis graph of parameter data that is preset and needs intensive monitoring for patients with different diseases. The parameter data that needs intensive monitoring includes one or more of following parameters: a perfusion index, a partial pressure of end-tidal carbon dioxide, an airway flow/pressure, a central venous pressure, a pulse pressure, a stroke volume, an intracranial pressure, and a bispectral index.

Optionally, each of the parameter data trend analysis areas in the multi-parameter analysis report is associated with each parameter analysis page in the report attachment. In response to clicking on a certain data trend analysis area on a screen displaying the multi-parameter analysis report, the multi-parameter analysis report automatically jumps to a corresponding parameter analysis page, to allow a user to view page by page the measurement statistical values, the analysis statistical charts, the waveform data, the numerical data, and marked event data, and perform a zoom in operation, a zoom out operation, or a scrolling operation on the screen for viewing, and select a range of data for viewing.

Optionally, after the multi-parameter analysis report and the report attachment are generated, the client with the first mode permission and the client with the second mode permission are also configured to transmit the multi-parameter analysis report, the report attachment, and the unique identifier index to an associated medical staff terminal.

Optionally, the system also includes a medical staff terminal, configured to transmit a report acquisition instruction to the application server. The report acquisition instruction includes a unique identifier index of a patient or a statistical parameter.

The application server is also configured to receive the report acquisition instruction transmitted by the medical staff terminal. When including the unique identifier index, the application server retrieves a multi-parameter analysis report and a report attachment according to the unique identifier index, and transmits the multi-parameter analysis report and the report attachment to the medical staff terminal; and
when including the statistical parameter, the application server retrieves a multi-parameter analysis report and a report attachment that satisfy the statistical parameter, and transmits the multi-parameter analysis report and the report attachment to the medical staff terminal.

Optionally, the application server is also configured to receive a report archiving instruction transmitted by a client or a medical staff terminal with a report archiving permission. The report archiving instruction includes the unique identifier index. The application server retrieves a multi-parameter analysis report and a report attachment according to the unique identifier index, and transmits the multi-parameter analysis report and the report attachment to a hospital information management system, an electronic medical record system, or a critical clinical information system, to store the multi-parameter analysis report and the report attachment in a patient electronic medical record.

A specific implementation process of the device embodiment may refer to the foregoing method embodiment, which is not detailed herein. Since this embodiment has the same principle as the foregoing method embodiment, the system has the corresponding technical effects of the foregoing method embodiment.

### Embodiment 3

This embodiment describes an actual application process of the foregoing method embodiment and system embodiment, which does not describe part of the claimed invention.

According to different application scenarios and requirements of users, devices such as a client, an application server, a database server, a medical staff terminal, and a printer, as well as a system management software, a data analysis report generation software, a mobile service software, a data management software may be combined to form a client/server (C/S) architecture, a browser/server (B/S) architecture, or a local client architecture, etc., so that a huge amount of multi-parameter data from various departments of a hospital can be centrally processed to generate a multi-parameter analysis report.

The application server is installed with the system management software, configured to control system operation. The medical staff terminal is installed with the data management software, configured to receive report files and perform data interaction. The desktop computer is installed with the data analysis report generation software or a browser. The mobile device is installed with the mobile service software or a browser, configured to support the client device to operate in systems under different architectures. The mobile device includes at least a tablet computer, a mobile computing device, a handheld mobile terminal, and a smartphone.

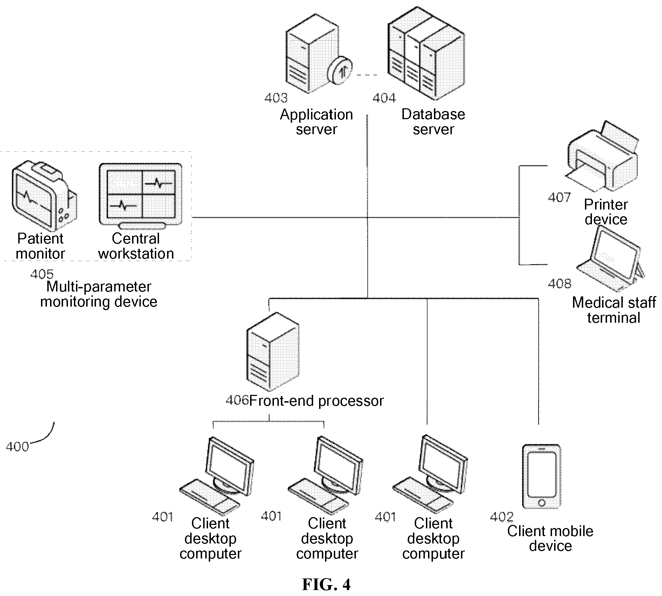

As shown in FIG. 4, a system 400 for generating a data analysis report of a multi-parameter monitoring device uses the C/S architecture, including: a client desktop computer 401 (the desktop computer is installed with the data analysis report generation software, and has the first mode permission), a client mobile device 402 (the mobile device is installed with the mobile service software that can transmit an instruction to the application server, and has the second mode permission), an application server 403, a database server 404, a multi-parameter monitoring device 405, a front-end processor 406, a printer device 407, and a medical staff terminal device 408. The front-end processor is configured to buffer the multi-parameter data. The printer device is configured to print the generated multi-parameter analysis report and report attachment. The functions of remaining devices refer to the foregoing embodiments.

In the C/S architecture, each device has a following interaction process.

Step 1. The application server receives the multi-parameter data transmitted in real time by the multi-parameter monitoring device, the multi-parameter data including patient information, waveform data, and numerical data; and stores the received multi-parameter data in the database server.

Specifically, the received multi-parameter data is stored in a second storage area (namely a data storage area) of the database server. In addition, a unique identifier of the multi-parameter data is configured and stored in a first storage area (namely an index storage area) of the database server, and a unique identifier index is established based on the unique identifier.

Step 2. The application server receives a report generation instruction transmitted by the client. The instruction includes a client identifier, which is configured for indicating a permission of the client. In this embodiment, when the client identifier indicates that the client is the desktop computer, it represents that the client has the first mode permission; and when the client identifier indicates that the client is the client mobile device, it represents that the client has the second mode permission. The client device identifier is configured for distinguishing the client is the desktop computer or the mobile device.

Step 3. The application server determines whether it has stored the client device identifier. If the client device identifier has not been stored, the application server transmits feedback information that the client does not have the report generation function to the client; and otherwise, the application server performs the following operations.

If the client device identifier is the desktop computer, the application server transmits the multi-parameter data to the client, and the desktop computer downloads the multi-parameter data to a local storage for calculation processing. The measurement statistical values, the time series identifiers, the event identifiers, and the patient basic information obtained by mapping the unique identifier index are imported in the multi-parameter analysis report framework, to generate the multi-parameter analysis report. The measurement statistical values, the analysis statistical charts, the time series identifiers, the event identifiers, as well as the multi-parameter waveform data and the numerical data are imported in the multi-parameter analysis report attachment framework, to generate the multi-parameter analysis report attachment. In addition, the client desktop computer marks the multi-parameter analysis report and the attachment according to the unique identifier of the data, and stores the multi-parameter analysis report and the attachment in a report file storage area (namely the third storage area) of the database server.

If the client device identifier is the mobile device, the application server processes the multi-parameter data, to generate the multi-parameter analysis report and the attachment in the above manner. The application server marks the multi-parameter analysis report and the attachment according to the unique identifier of the data, and stores the multi-parameter analysis report and the attachment in the report file storage area of the database server.

In addition, if the client device identifier is the desktop computer, the client desktop computer also controls the application server to transmit the report files to the medical staff terminal device for viewing, and at the same time, controls the printer to output the multi-parameter analysis report and the attachment.

If the client device identifier is the mobile device, the client mobile device transmits an instruction to the application server, to control the application server to transmit the report files to the medical staff terminal device for viewing. The client mobile device downloads the report files to a memory, and controls the printer to output the multi-parameter analysis report and the attachment.

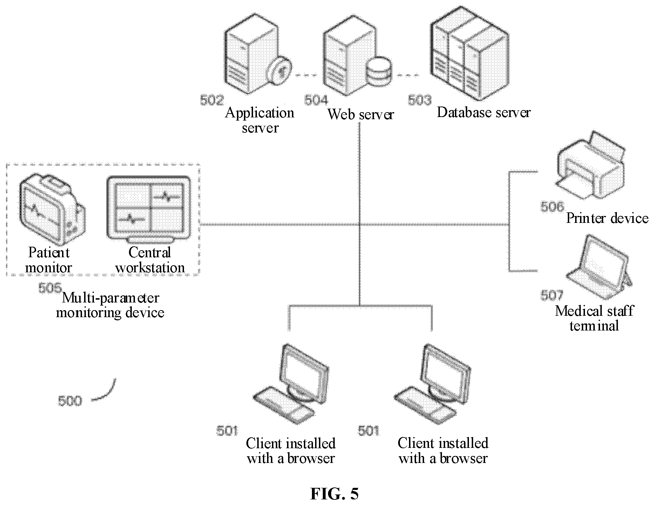

As shown in FIG. 5, a system 500 for generating a data analysis report of a multi-parameter monitoring device uses the B/S architecture, including: a client device 501 installed with a browser, an application server 502, a database server 503, a WEB server 504, a multi-parameter monitoring device 505, a printer device 506, and a medical staff terminal device 507.

In the B/S architecture, each device has a following interaction process.

Step S1. This step is the same as above, which is not detailed herein.

Step S2. The application server receives a report generation instruction that is transmitted by the client browser and successfully authenticated by the WEB server. The report generation instruction includes a client browser identifier, and the permission of the client indicated by the client browser identifier is the second mode permission.

Step S3. In response to determining that the permission of the client is the second mode permission, the application server processes the multi-parameter data, to generate the multi-parameter analysis report and the attachment in the above manner. The application server marks the multi-parameter analysis report and the attachment according to the unique identifier of the data, and stores the multi-parameter analysis report and the attachment in the report file storage area of the database server.

In addition, after the multi-parameter analysis report and the attachment are generated, the client browser also transmits an instruction to the WEB server, to control the application server to transmit the report files to the medical staff terminal device for viewing. The client browser also downloads the report files to a memory, and controls the printer to output the multi-parameter analysis report and the attachment.

It should be noted that due to the characteristics of the B/S architecture, transmission and interaction of uplink and downlink data information between the client browser and the application server need to be performed by the WEB server.

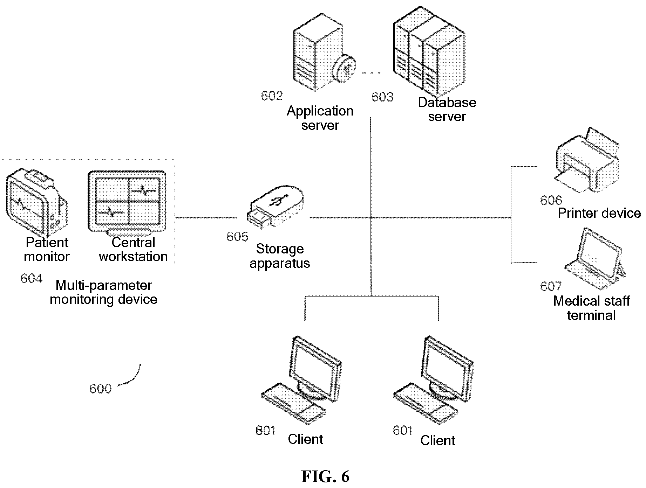

As shown in FIG. 6, a system 600 for generating a data analysis report of a multi-parameter monitoring device uses the local client architecture, including: a client device 601, an application server 602, a database server 603, a multi-parameter monitoring device 604, a mobile storage apparatus 605, a printer device 606, and a medical staff terminal device 607.

In this system, the mobile storage apparatus is connected to an interface of the multi-parameter monitoring device, to download the multi-parameter data. The client is connected to an interface of the mobile storage apparatus, to read the multi-parameter data, and upload the multi-parameter data to the application server. The subsequent processing process may refer to the foregoing method.

In addition, when the client device fails to connect to the application server, the report files may be generated in the following way:
The client device stores the multi-parameter data in a memory of the client device, after reading the multi-parameter data from the interface of the mobile storage apparatus.
The client device processes the multi-parameter data, to generate the multi-parameter analysis report and the attachment, and stores the multi-parameter analysis report and the attachment in a memory of the client device.

In this case, the client device is installed with a software program configured for generating the multi-parameter analysis report and the attachment.

From above, the multi-parameter analysis report and the attachment can be generated in different system architectures, which greatly expands application scenarios of the present disclosure, thereby satisfying various application scenarios and requirements of hospitals.

Those skilled in the art can understand that relevant hard wares can be instructed through computer programs to implement all or part of processes in the method according to the above examples, and the programs can be stored in a computer-readable storage medium. The computer-readable storage medium may be a magnetic disk, an optical disk, a read-only memory (ROM), a random-access memory (RAM), or the like.

## Claims

1. A method for generating a data analysis report of a multi-parameter monitoring device, **characterized in that**, comprising:
receiving, by an application server (403, 502, 602), multi-parameter data transmitted in real time by the multi-parameter monitoring device (405, 505, 604), and storing the received multi-parameter data in a database server (404, 503, 603);
receiving, by the application server, a report generation instruction transmitted by a client (401, 501, 601), the instruction comprising a client identifier that is configured for indicating a permission of the client; and
in a case that the permission of the client is a first mode permission, transmitting, by the application server, the multi-parameter data to the client, to allow the client to process the multi-parameter data, so as to generate a multi-parameter analysis report (200) and a report attachment; and in a case that the permission of the client is a second mode permission, processing, by the application server, the multi-parameter data to generate the multi-parameter analysis report and the report attachment, and transmitting the multi-parameter analysis report and the report attachment to the client;
wherein after the receiving, by an application server, multi-parameter data transmitted in real time by the multi-parameter monitoring device, the method further comprises: configuring a unique identifier for the multi-parameter data;
storing the configured unique identifier in a first storage area of the database server, and establishing a unique identifier index based on the unique identifier;
storing the received multi-parameter data in a second storage area of the database server, and marking the multi-parameter data according to the unique identifier of the multi-parameter data; and
storing the generated multi-parameter analysis report and the report attachment in a third storage area of the database server, and marking the generated multi-parameter analysis report and the report attachment according to the unique identifier of the multi-parameter data;
wherein the unique identifier index established by the first storage area has a mutual mapping relationship with the second storage area and the third storage area, respectively, to provide unified management of information stored in the first storage area, the second storage area, and the third storage area that is associated by the unique identifier;
wherein after the receiving, by an application server, multi-parameter data transmitted in real time by the multi-parameter monitoring device, the method further comprises:
counting, by the application server, a time length of the received multi-parameter data;
in a case that the time length of the multi-parameter data reaches a predetermined time length, transmitting the unique identifier index of the multi-parameter data of the predetermined time length to the client;
in a case that the time length of the multi-parameter data is less than the predetermined time length and no longer increases, transmitting the unique identifier index of the multi-parameter data whose time length is less than the predetermined time length and no longer increases to the client; and
transmitting, by the client, the report generation instruction to the application server according to the received unique identifier index;
wherein the report generation instruction further comprises a data time period parameter for report generation; and
the method further comprises: determining, by the application server, to-be-processed multi-parameter data of a corresponding time period length based on the data time period parameter for report generation, and generating the multi-parameter analysis report and the report attachment based on the determined multi-parameter data of the corresponding time period length.

2. The method for generating a data analysis report of a multi-parameter monitoring device according to claim 1, wherein the multi-parameter data comprises patient information, waveform data, and numerical data;
wherein the generating a multi-parameter analysis report and a report attachment comprises:
processing the multi-parameter data, to obtain measurement statistical values, analysis statistical charts, time series identifiers, and event identifiers, the measurement statistical values comprising an average value, a maximum value, a minimum value, and a variability analysis value;
importing the measurement statistical values, the time series identifiers, the event identifiers, and patient basic information obtained by mapping the unique identifier index into a multi-parameter analysis report framework, to generate the multi-parameter analysis report; and
importing the measurement statistical values, the analysis statistical charts, the time series identifiers, the event identifiers, the waveform data, and the numerical data into a multi-parameter analysis report attachment framework, to generate the report attachment;
wherein the multi-parameter analysis report is configured for displaying a change trend of each parameter and a data analysis conclusion, and the report attachment is configured for displaying an analysis report of each parameter comprising the waveform data and the numerical data;
the multi-parameter analysis report framework comprises at least a patient basic information area (201);
and further comprises:
one or more of following parameter data trend analysis areas: a heart rate data trend analysis area (202), a blood pressure data trend analysis area (203), a respiratory rate data trend analysis area (204), a blood oxygen saturation level data trend analysis area (205), a body temperature data trend analysis area (206), and a designated data trend analysis area (207); and
a data analysis conclusion area (208);
the report generation instruction further comprises a setting parameter of the designated data trend analysis area, according to which the application server sets content of the designated data trend analysis area; and
the report generation instruction further comprises a setting parameter of the data analysis conclusion area, according to which the application server sets content of the data analysis conclusion area;
wherein each of the parameter data trend analysis areas comprises an ordinate set as a numerical scale and an abscissa set as a time scale, configured for displaying a state in a unit time and a change process of each parameter, and comprises at least the measurement statistical values, the time series identifiers, and the event identifiers of each parameter;
wherein the designated data trend analysis area is configured for displaying a trend analysis graph of parameter data that is preset and needs intensive monitoring for patients with different diseases;
wherein the parameter data that needs intensive monitoring comprises one or more of following parameters: a perfusion index, a partial pressure of end-tidal carbon dioxide, an airway flow/pressure, a central venous pressure, a pulse pressure, a stroke volume, an intracranial pressure, and a bispectral index;
each of the parameter data trend analysis areas in the multi-parameter analysis report is associated with each parameter analysis page in the report attachment, and in response to clicking on a certain data trend analysis area on a screen displaying the multi-parameter analysis report, the multi-parameter analysis report automatically jumps to a corresponding parameter analysis page, to allow a user to view page by page the measurement statistical values, the analysis statistical charts, the waveform data, the numerical data, and marked event data, and perform a zoom in operation, a zoom out operation, or a scrolling operation on the screen for viewing, and select a range of data for viewing.

3. The method for generating a data analysis report of a multi-parameter monitoring device according to claim 1, wherein,
the client with the first mode permission and the client with the second mode permission transmit the multi-parameter analysis report, the report attachment, and the unique identifier index to an associated medical staff terminal (507), after the multi-parameter analysis report and the report attachment are generated;
wherein the method further comprises:
receiving, by the application server, a report acquisition instruction transmitted by the medical staff terminal, the report acquisition instruction comprising a unique identifier index of a patient or a statistical parameter;
in a case that the unique identifier index is comprised, retrieving, by the application server, a multi-parameter analysis report and a report attachment according to the unique identifier index, and transmitting the multi-parameter analysis report and the report attachment to the medical staff terminal; and
in a case that the statistical parameter is comprised, retrieving, by the application server, a multi-parameter analysis report and a report attachment that satisfy the statistical parameter, and transmitting the multi-parameter analysis report and the report attachment to the medical staff terminal.

4. The method for generating a data analysis report of a multi-parameter monitoring device according to claim 3, wherein the method further comprises:
receiving, by the application server, a report archiving instruction transmitted by a client or a medical staff terminal with a report archiving permission, the report archiving instruction comprising the unique identifier index; and retrieving, by the application server, a multi-parameter analysis report and a report attachment according to the unique identifier index, and transmitting the multi-parameter analysis report and the report attachment to a hospital information management system, an electronic medical record system, or a critical clinical information system, to store the multi-parameter analysis report and the report attachment in a patient electronic medical record.

5. The method for generating a data analysis report of a multi-parameter monitoring device according to claim 1, wherein the receiving, by the application server, a report generation instruction transmitted by a client further comprises:
receiving, by the application server, the report generation instruction that is transmitted by a client browser and successfully authenticated by a world wide web (WEB) server (504), the report generation instruction comprising a client browser identifier, and the permission of the client indicated by the client browser identifier being the second mode permission.

6. The method for generating a data analysis report of a multi-parameter monitoring device according to claim 1, wherein the receiving, by an application server, multi-parameter data transmitted in real time by the multi-parameter monitoring device comprises:
connecting, by a mobile storage apparatus (607), to an interface of the multi-parameter monitoring device, to download the multi-parameter data; and
connecting, by the client, to an interface of the mobile storage apparatus, to upload read multi-parameter data to the application server.

7. A system for generating a data analysis report of a multi-parameter monitoring device, **characterized in that**, comprising:
one or more clients (401, 501, 601), configured to transmit a report generation instruction to an application server (403, 502, 602), the report generation instruction comprising a client identifier that is configured for indicating a permission of the client;
the application server, configured to receive multi-parameter data transmitted in real time by the multi-parameter monitoring device (405, 505, 604); and further configured to receive the report generation instruction transmitted by the client, in a case that the permission of the client is a first mode permission, transmit the multi-parameter data to the client, to allow the client to process the multi-parameter data, so as to generate a multi-parameter analysis report and a report attachment, and in a case that the permission of the client is a second mode permission, process the multi-parameter data to generate the multi-parameter analysis report (200) and the report attachment, and transmit the multi-parameter analysis report and the report attachment to the client; and
a database server (404, 503, 603), configured to store the multi-parameter data received by the application server;
wherein,
the application server is further configured to configure a unique identifier for the multi-parameter data after receiving the multi-parameter data transmitted in real time by the multi-parameter monitoring device;
the database server comprises a first storage area, a second storage area, and a third storage area; wherein,
the first storage area of the database server is configured to store the configured unique identifier, and establish a unique identifier index based on the unique identifier;
the second storage area of the database server is configured to store the multi-parameter data received by the application server, and mark the multi-parameter data according to the unique identifier of the multi-parameter data; and
the third storage area of the database server is configured to store the generated multi-parameter analysis report and the report attachment, and mark the generated multi-parameter analysis report and the report attachment according to the unique identifier of the multi-parameter data;
wherein the unique identifier index established by the first storage area has a mutual mapping relationship with the second storage area and the third storage area, respectively, to provide unified management of information stored in the first storage area, the second storage area, and the third storage area that is associated by the unique identifier;
wherein,
the application server is further configured to count a time length of the received multi-parameter data, after receiving the multi-parameter data transmitted in real time by the multi-parameter monitoring device;
in a case that the time length of the received multi-parameter data reaches a predetermined time length, transmit the unique identifier index of the multi-parameter data of the predetermined time length to the client; and
in a case that the time length of the multi-parameter data is less than the predetermined time length and no longer increases, transmit the unique identifier index of the multi-parameter data whose time length is less than the predetermined time length and no longer increases to the client; and
the client is further configured to transmit the report generation instruction to the application server according to the received unique identifier index;
wherein the report generation instruction further comprises a data time period parameter for report generation; and
the application server is further configured to determine to-be-processed multi-parameter data of a corresponding time period length based on the data time period parameter for report generation, and generate the multi-parameter analysis report and the report attachment based on the determined multi-parameter data of the corresponding time period length.

8. The system for generating a data analysis report of a multi-parameter monitoring device according to claim 7, wherein the multi-parameter data comprises patient information, waveform data, and numerical data;
wherein the generation of the multi-parameter analysis report and the report attachment comprises:
processing the multi-parameter data, to obtain measurement statistical values, analysis statistical charts, time series identifiers, and event identifiers, the measurement statistical values comprising an average value, a maximum value, a minimum value, and a variability analysis value;
importing the measurement statistical values, the time series identifiers, the event identifiers, and patient basic information obtained by mapping the unique identifier index into a multi-parameter analysis report framework, to generate the multi-parameter analysis report; and
importing the measurement statistical values, the analysis statistical charts, the time series identifiers, the event identifiers, the waveform data, and the numerical data into a multi-parameter analysis report attachment framework, to generate the report attachment;
wherein the multi-parameter analysis report is configured for displaying a change trend of each parameter and a data analysis conclusion, and the report attachment is configured for displaying an analysis report of each parameter comprising the waveform data and the numerical data;
the multi-parameter analysis report framework comprises at least a patient basic information area (201);
and further comprises:
one or more of following parameter data trend analysis areas: a heart rate data trend analysis area (202), a blood pressure data trend analysis area (203), a respiratory rate data trend analysis area (204), a blood oxygen saturation level data trend analysis area (205), a body temperature data trend analysis area (206), and a designated data trend analysis area (207); and
a data analysis conclusion area (208);
the report generation instruction further comprises a setting parameter of the designated data trend analysis area, according to which the application server sets content of the designated data trend analysis area; and
the report generation instruction further comprises a setting parameter of the data analysis conclusion area, according to which the application server sets content of the data analysis conclusion area;
wherein each of the parameter data trend analysis areas comprises an ordinate set as a numerical scale and an abscissa set as a time scale, configured for displaying a state in a unit time and a change process of each parameter, and comprises at least the measurement statistical values, the time series identifiers, and the event identifiers of each parameter;
wherein the designated data trend analysis area is configured for displaying a trend analysis graph of parameter data that is preset and needs intensive monitoring for patients with different diseases;
wherein the parameter data that needs intensive monitoring comprises one or more of following parameters: a perfusion index, a partial pressure of end-tidal carbon dioxide, an airway flow/pressure, a central venous pressure, a pulse pressure, a stroke volume, an intracranial pressure, and a bispectral index;
each of the parameter data trend analysis areas in the multi-parameter analysis report is associated with each parameter analysis page in the report attachment, and in response to clicking on a certain data trend analysis area on a screen displaying the multi-parameter analysis report, the multi-parameter analysis report automatically jumps to a corresponding parameter analysis page, to allow a user to view page by page the measurement statistical values, the analysis statistical charts, the waveform data, the numerical data, and marked event data, and perform a zoom in operation, a zoom out operation, or a scrolling operation on the screen for viewing, and select a range of data for viewing.

9. The system for generating a data analysis report of a multi-parameter monitoring device according to claim 7, wherein,
the client with the first mode permission and the client with the second mode permission are further configured to transmit the multi-parameter analysis report, the report attachment, and the unique identifier index to an associated medical staff terminal (507), after the multi-parameter analysis report and the report attachment are generated;
the system further comprises a medical staff terminal, configured to transmit a report acquisition instruction to the application server, the report acquisition instruction comprising a unique identifier index of a patient or a statistical parameter; and
the application server is further configured to receive the report acquisition instruction transmitted by the medical staff terminal, in a case that the unique identifier index is comprised, retrieve a multi-parameter analysis report and a report attachment according to the unique identifier index, and transmit the multi-parameter analysis report and the report attachment to the medical staff terminal; and
in a case that the statistical parameter is comprised, retrieve a multi-parameter analysis report and a report attachment that satisfy the statistical parameter, and transmit the multi-parameter analysis report and the report attachment to the medical staff terminal.

10. The system for generating a data analysis report of a multi-parameter monitoring device according to claim 9, wherein,
the application server is further configured to receive a report archiving instruction transmitted by a client or a medical staff terminal with a report archiving permission, the report archiving instruction comprising the unique identifier index, retrieve a multi-parameter analysis report and a report attachment according to the unique identifier index, and transmit the multi-parameter analysis report and the report attachment to a hospital information management system, an electronic medical record system, or a critical clinical information system, to store the multi-parameter analysis report and the report attachment in a patient electronic medical record.

11. The system for generating a data analysis report of a multi-parameter monitoring device according to claim 7, wherein the system further comprises a WEB server (504); and
the application server is configured to receive the report generation instruction that is transmitted by a client browser and successfully authenticated by the WEB server, the report generation instruction comprising a client browser identifier, and the permission of the client indicated by the client browser identifier being the second mode permission;
the system further comprises a mobile storage apparatus;
the application server is configured to receive the multi-parameter data transmitted by the multi-parameter monitoring device, comprising:
a mobile storage apparatus (607) connecting to an interface of the multi-parameter monitoring device, to download the multi-parameter data; and
the client connecting to an interface of the mobile storage apparatus, to upload read multi-parameter data to the application server.

## Patentansprüche

1. Ein Verfahren zum Generieren eines Datenanalyseberichts eines Multiparameter-Überwachungsgeräts, **dadurch gekennzeichnet, dass** umfassend:
Empfangen von Multiparameterdaten, die in Echtzeit von der Multiparameter-Überwachungsgeräte (405, 505, 604) übertragen werden, durch dem Anwendungsserver (403, 502, 602) und Speichern der empfangenen Multiparameterdaten in den Datenbankserver (404, 503, 603);
Empfangen einer vom Client (401, 501, 601) übertragenen Anweisungen zur Berichterstellung durch den Anwendungsserver, wobei die Anweisung eine Clientkennung umfasst, die zum Anzeigen einer Berechtigung des Clients konfiguriert ist; und
wenn die Berechtigung des Clients eine Berechtigung des ersten Modus ist, Übertragen der Multiparameterdaten durch den Anwendungsserver an den Client, um dem Client die Verarbeitung der Multiparameterdaten zu gestatten, um einen Multiparameteranalysebericht (200) und einen Berichtsanhang zu erzeugen; und wenn die Berechtigung des Clients eine Berechtigung des zweiten Modus ist, Verarbeiten der Multiparameterdaten durch den Anwendungsserver, um den Multiparameteranalysebericht und den Berichtsanhang zu erzeugen, und Übertragen des Multiparameteranalyseberichts und des Berichtsanhangs an den Client;
nach dem Empfang der in Echtzeit vom Multiparameter-Überwachungsgerät übertragenen Multiparameter-Daten durch einen Anwendungsserver weiterhin Folgendes umfasst:
Konfigurieren einer eindeutigen Kennung für die Multiparameter-Daten;
Speichern der konfigurierten eindeutigen Kennung in einem ersten Speicherbereich des Datenbankservers und Erstellen eines eindeutigen Kennungsindex basierend auf der eindeutigen Kennung;
Speichern der empfangenen Multiparameterdaten in einem zweiten Speicherbereich des Datenbankservers und Markieren der Multiparameterdaten entsprechend der eindeutigen Kennung der Multiparameterdaten; und
Speichern des erstellten Multiparameter-Analyseberichts und des Berichtsanhangs in einem dritten Speicherbereich des Datenbankservers und Markieren des erstellten Multiparameter-Analyseberichts und des Berichtsanhangs entsprechend der eindeutigen Kennung der Multiparameterdaten;
wobei der durch den ersten Speicherbereich erstellte eindeutige Kennungsindex eine gegenseitige Zuordnungsbeziehung mit dem zweiten Speicherbereich bzw. dem dritten Speicherbereich aufweist, um eine einheitliche Verwaltung der im ersten Speicherbereich, dem zweiten Speicherbereich und dem dritten Speicherbereich gespeicherten Informationen bereitzustellen, die durch die eindeutige Kennung verknüpft sind;
nach dem Empfangen der in Echtzeit vom Multiparameter-Überwachungsgerät übertragenen Multiparameter-Daten durch einen Anwendungsserver weiterhin umfasst:
Zählen der Zeitdauer der empfangenen Multiparameterdaten durch den Anwendungsserver;
falls die Zeitdauer der Multiparameterdaten eine vorgegebene Zeitdauer erreicht, Übertragen des eindeutigen Identifizierungsindex der Multiparameterdaten der vorgegebenen Zeitdauer an den Client;
falls die Zeitdauer der Multiparameterdaten kleiner als die vorgegebene Zeitdauer ist und nicht mehr zunimmt, Übertragen des eindeutigen Kennungsindex der Multiparameterdaten, deren Zeitdauer kleiner als die vorgegebene Zeitdauer ist und nicht mehr zunimmt, an den Client; und
Übertragen der Anweisung zur Berichterstellung durch den Client an den Anwendungsserver gemäß dem empfangenen eindeutigen Identifikationsindex;
wobei die Anweisung zur Berichtsgenerierung außerdem einen Datenzeitraumparameter für die Berichtsgenerierung umfasst;
und das Verfahren außerdem umfasst:
Bestimmen, durch den Anwendungsserver, von zu verarbeitenden Multiparameterdaten einer entsprechenden Zeitraumlänge basierend auf dem Datenzeitraumparameter für die Berichtsgenerierung, und Generieren des Multiparameter-Analyseberichts und des Berichtsanhangs basierend auf den bestimmten Multiparameterdaten der entsprechenden Zeitraumlänge.

2. Das Verfahren zum Generieren eines Datenanalyseberichts eines Multiparameter-Überwachungsgeräts gemäß Anspruch 1, wobei die Multiparameterdaten Patienteninformationen, Wellenformdaten und numerische Daten umfassen;
das Generieren eines Multiparameter-Analyseberichts und eines Berichtsanhangs umfasst:
Verarbeiten der Multiparameterdaten, um statistische Messwerte, statistische Analysediagramme, Zeitreihenkennungen und Ereigniskennungen zu erhalten, wobei die statistischen Messwerte einen Durchschnittswert, einen Maximalwert, einen Minimalwert und einen Variabilitätsanalysewert umfassen;
Importieren der statistischen Messwerte, der Zeitreihenkennungen, der Ereigniskennungen und der durch die Abbildung des eindeutigen Kennungsindex erhaltenen Patientengrundinformationen in ein Rahmenwerk für einen Multiparameteranalysebericht, um den Multiparameteranalysebericht zu erstellen; und
Importieren der statistischen Messwerte, der statistischen Analysediagramme, der Zeitreihenkennungen, der Ereigniskennungen, der Wellenformdaten und der numerischen Daten in ein Framework für den Anhang eines Mehrparameteranalyseberichts, um den Berichtsanhang zu generieren;
wobei der Multiparameter-Analysebericht zum Anzeigen eines Änderungstrends jedes Parameters und einer Schlussfolgerung aus der Datenanalyse konfiguriert ist und der Berichtsanhang zum Anzeigen eines Analyseberichts jedes Parameters konfiguriert ist, der die Wellenformdaten und die numerischen Daten umfasst;
der Rahmen für den Bericht zur Multiparameteranalyse mindestens einen Bereich (201) mit grundlegenden Patienteninformationen umfasst;
und weiterhin umfasst:
einen oder mehrere der folgenden Parameterdaten-Trendanalysebereiche: einen Herzfrequenzdaten-Trendanalysebereich (202), einen Blutdruckdaten-Trendanalysebereich (203), einen Atemfrequenzdaten-Trendanalysebereich (204), einen Blutsauerstoffsättigungsgrad-Trendanalysebereich (205), einen Körpertemperaturdaten-Trendanalysebereich (206) und einen ausgewiesenen Datentrendanalysebereich (207); und
ein Bereich (208) für Schlussfolgerungen zur Datenanalyse;
die Anweisung zur Berichterstellung ferner einen Einstellungsparameter für den angegebenen Datentrendanalysebereich umfasst, gemäß dem der Anwendungsserver den Inhalt des angegebenen Datentrendanalysebereichs einstellt;
die Anweisung zur Berichterstellung außerdem einen Einstellungsparameter für den Datenanalyse-Abschlussbereich umfasst, gemäß dem der Anwendungsserver den Inhalt des Datenanalyse-Abschlussbereichs einstellt;
jeder der Parameterdaten-Trendanalysebereiche eine Ordinate als numerische Skala und eine Abszisse als Zeitskala umfasst, die zum Anzeigen eines Zustands in einer Zeiteinheit und eines Änderungsprozesses jedes Parameters konfiguriert sind und mindestens die statistischen Messwerte, die Zeitreihenkennungen und die Ereigniskennungen jedes Parameters umfassen;
der bezeichnete Datentrendanalysebereich zum Anzeigen eines Trendanalysediagramms von Parameterdaten konfiguriert ist, die voreingestellt sind und für Patienten mit unterschiedlichen Krankheiten eine intensive Überwachung erfordern; wobei die Parameterdaten, die eine intensive Überwachung erfordern, einen oder mehrere der folgenden Parameter umfassen: einen Perfusionsindex, einen Partialdruck von endtidalem Kohlendioxid, einen Atemwegsfluss/-druck, einen zentralvenösen Druck, einen Pulsdruck, ein Schlagvolumen, einen intrakraniellen Druck und einen Bispektralindex;
jeder der Parameterdatentrendanalysebereiche im Multiparameter-Analysebericht jeder Parameteranalyseseite im Berichtsanhang zugeordnet ist, und beim Klicken auf einen bestimmten Datentrendanalysebereich auf einem Bildschirm, auf dem der Multiparameter-Analysebericht angezeigt wird, der Multiparameter-Analysebericht automatisch zu einer entsprechenden Parameteranalyseseite springt, um einem Benutzer das Anzeigen der statistischen Messwerte, der statistischen Analysediagramme, der Wellenformdaten, der numerischen Daten und der markierten Ereignisdaten seitenweise zu ermöglichen und zum Anzeigen eine Vergrößerungs-, Verkleinerungs- oder Bildlaufoperation auf dem Bildschirm durchzuführen und einen Datenbereich zum Anzeigen auszuwählen.

3. Das Verfahren zum Generieren eines Datenanalyseberichts eines Multiparameter-Überwachungsgeräts gemäß Anspruch 1, wobei der Client mit der Berechtigung des ersten Modus und der Client mit der Berechtigung des zweiten Modus den Multiparameter-Analysebericht, den Berichtsanhang und den eindeutigen Identifizierungsindex an ein zugehöriges medizinisches Personalterminal (507) übertragen, nachdem der Multiparameter-Analysebericht und der Berichtsanhang generiert wurden;
wobei das Verfahren weiterhin umfasst:
Empfangen einer vom medizinischen Personalterminal übertragenen Anweisung zum Abrufen eines Berichts durch den Anwendungsserver, wobei die Anweisung zum Abrufen des Berichts einen eindeutigen Identifikationsindex eines Patienten oder einen statistischen Parameter umfasst;
falls der eindeutige Kennungsindex enthalten ist, Abrufen eines Multiparameter-Analyseberichts und eines Berichtsanhangs durch den Anwendungsserver entsprechend dem eindeutigen Kennungsindex und Übertragen des Multiparameter-Analyseberichts und des Berichtsanhangs an das Medizinisches Personalterminal; und
falls der statistische Parameter enthalten ist, Abrufen eines Multiparameter-Analyseberichts und eines Berichtsanhangs durch den Anwendungsserver, die den statistischen Parameter erfüllen, und Übertragen des Multiparameter-Analyseberichts und des Berichtsanhangs an das Medizinisches Personalterminal.

4. Das Verfahren zum Generieren eines Datenanalyseberichts eines Multiparameter-Überwachungsgeräts nach Anspruch 3, wobei das Verfahren ferner umfasst: Empfangen einer von einem Client oder einem medizinischen Personalterminal mit einer Berichtsarchivierungsberechtigung übertragenen Berichtsarchivierungsanweisung durch den Anwendungsserver, wobei die Berichtsarchivierungsanweisung den eindeutigen Kennungsindex umfasst; und Abrufen eines Multiparameter-Analyseberichts und eines Berichtsanhangs durch den Anwendungsserver gemäß dem eindeutigen Kennungsindex und Übertragen des Multiparameter-Analyseberichts und des Berichtsanhangs an ein Krankenhausinformationsmanagementsystem, ein elektronisches Patientenaktensystem oder ein kritisches klinisches Informationssystem, um den Multiparameter-Analysebericht und den Berichtsanhang in einer elektronischen Patientenakte zu speichern.

5. Das Verfahren zum Generieren eines Datenanalyseberichts eines Multiparameter-Überwachungsgeräts gemäß Anspruch 1, wobei das Empfangen einer von einem Client übertragenen Anweisung zur Berichtserstellung durch den Anwendungsserver weiterhin umfasst: Empfangen der Anweisung zur Berichtserstellung durch den Anwendungsserver, die von einem Client-Browser übertragen und von einem World Wide Web (WEB)-Server (504) erfolgreich authentifiziert wurde, wobei die Anweisung zur Berichtserstellung eine Client-Browser-Kennung umfasst und die durch die Client-Browser-Kennung angegebene Berechtigung des Clients die Berechtigung des zweiten Modus ist.

6. Das Verfahren zum Erzeugen eines Datenanalyseberichts eines Multiparameter-Überwachungsgeräts gemäß Anspruch 1, wobei das Empfangen von Multiparameter-Daten, die in Echtzeit vom Multiparameter-Überwachungsgerät übertragen werden, durch einen Anwendungsserver Folgendes umfasst:
Verbinden eines mobilen Speichergeräts (607) mit einer Schnittstelle des Multiparameter-Überwachungsgeräts, um die Multiparameterdaten herunterzuladen; und
Verbinden des Clients mit einer Schnittstelle des mobilen Speichergeräts, um gelesene Multiparameterdaten auf den Anwendungsserver hochzuladen.

7. Ein System zum Generieren eines Datenanalyseberichts eines Multiparameter-Überwachungsgeräts, umfassend:
einen oder mehrere Clients (401, 501, 601), die dazu konfiguriert sind, eine Anweisung zur Berichterstellung an einen Anwendungsserver (403, 502, 602) zu übertragen, wobei die Anweisung zur Berichterstellung eine Clientkennung umfasst, die dazu konfiguriert ist, eine Berechtigung des Clients anzuzeigen;
der Anwendungsserver ist so konfiguriert, dass er in Echtzeit von der Multiparameter-Überwachungsgeräte (405, 505, 604) übertragene Multiparameterdaten empfängt;
und ferner so konfiguriert ist, dass er die vom Client übertragene Anweisung zur Berichterstellung empfängt, falls die Berechtigung des Clients eine Berechtigung des ersten Modus ist, die Multiparameterdaten an den Client überträgt, um dem Client die Verarbeitung der Multiparameterdaten zu ermöglichen, um einen Multiparameter-Analysebericht und einen Berichtsanhang zu erzeugen, und falls die Berechtigung des Clients eine Berechtigung des zweiten Modus ist, die Multiparameterdaten verarbeitet, um den Multiparameter-Analysebericht und den Berichtsanhang zu erzeugen, und den Multiparameter-Analysebericht (200) und den Berichtsanhang an den Client überträgt; und
ein Datenbankserver (404, 503, 603), der zum Speichern der vom Anwendungsserver empfangenen Multiparameterdaten konfiguriert ist;
der Anwendungsserver ferner so konfiguriert ist, dass er nach dem Empfang der in Echtzeit vom Multiparameter-Überwachungsgerät übertragenen Multiparameter-Daten eine eindeutige Kennung für die Multiparameter-Daten konfiguriert;
der Datenbankserver einen ersten Speicherbereich, einen zweiten Speicherbereich und einen dritten Speicherbereich umfasst; wobei
der erste Speicherbereich des Datenbankservers so konfiguriert ist, dass er die konfigurierte eindeutige Kennung speichert und einen eindeutigen Kennungsindex basierend auf der eindeutigen Kennung erstellt;
der zweite Speicherbereich des Datenbankservers so konfiguriert ist, dass er die vom Anwendungsserver empfangenen Multiparameterdaten speichert und die Multiparameterdaten entsprechend der eindeutigen Kennung der Multiparameterdaten markiert;
der dritte Speicherbereich des Datenbankservers so konfiguriert ist, dass er den generierten Multiparameter-Analysebericht und den Berichtsanhang speichert und den generierten Multiparameter-Analysebericht und den Berichtsanhang entsprechend der eindeutigen Kennung der Multiparameterdaten markiert;
wobei der durch den ersten Speicherbereich erstellte eindeutige Kennungsindex eine wechselseitige Abbildungsbeziehung mit dem zweiten Speicherbereich bzw. dem dritten Speicherbereich aufweist, um eine einheitliche Verwaltung der im ersten Speicherbereich, im zweiten Speicherbereich und im dritten Speicherbereich gespeicherten Informationen bereitzustellen, die durch die eindeutige Kennung verknüpft sind;
der Anwendungsserver ferner dazu konfiguriert ist, die Zeitdauer der empfangenen Multiparameterdaten zu zählen, nachdem er die in Echtzeit vom Multiparameter-Überwachungsgerät übertragenen Multiparameterdaten empfangen hat;
falls die Zeitdauer der empfangenen Multiparameterdaten eine vorgegebene Zeitdauer erreicht, Übertragen des eindeutigen Identifizierungsindex der Multiparameterdaten der vorgegebenen Zeitdauer an den Client; und
falls die Zeitdauer der Multiparameterdaten kleiner als die vorgegebene Zeitdauer ist und nicht mehr zunimmt, Übertragen des eindeutigen Kennungsindex der Multiparameterdaten, deren Zeitdauer kleiner als die vorgegebene Zeitdauer ist und nicht mehr zunimmt, an den Client; und
der Client ist außerdem so konfiguriert, dass er die Anweisung zur Berichterstellung entsprechend dem empfangenen eindeutigen Kennungsindex an den Anwendungsserver überträgt;
die Anweisung zur Berichtsgenerierung außerdem einen Datenzeitraumparameter für die Berichtsgenerierung umfasst;
und der Anwendungsserver außerdem so konfiguriert ist, dass er zu verarbeitende Multiparameterdaten einer entsprechenden Zeitraumlänge basierend auf dem Datenzeitraumparameter für die Berichtsgenerierung ermittelt und den Multiparameter-Analysebericht und den Berichtsanhang basierend auf den ermittelten Multiparameterdaten der entsprechenden Zeitraumlänge generiert.

8. Das System zum Generieren eines Datenanalyseberichts eines Multiparameter-Überwachungsgeräts gemäß Anspruch 7, wobei die Multiparameterdaten Patienteninformationen, Wellenformdaten und numerische Daten umfassen;
das Generieren des Multiparameter-Analyseberichts und des Berichtsanhangs umfasst:
Verarbeiten der Multiparameterdaten, um statistische Messwerte, statistische Analysediagramme, Zeitreihenkennungen und Ereigniskennungen zu erhalten, wobei die statistischen Messwerte einen Durchschnittswert, einen Maximalwert, einen Minimalwert und einen Variabilitätsanalysewert umfassen;
Importieren der statistischen Messwerte, der Zeitreihenkennungen, der Ereigniskennungen und der durch die Abbildung des eindeutigen Kennungsindex erhaltenen Patientengrundinformationen in ein Rahmenwerk für einen Multiparameteranalysebericht, um den Multiparameteranalysebericht zu erstellen; und
Importieren der statistischen Messwerte, der statistischen Analysediagramme, der Zeitreihenkennungen, der Ereigniskennungen, der Wellenformdaten und der numerischen Daten in ein Framework für den Anhang eines Mehrparameteranalyseberichts, um den Berichtsanhang zu generieren;
wobei der Multiparameter-Analysebericht zum Anzeigen eines Änderungstrends jedes Parameters und einer Schlussfolgerung aus der Datenanalyse konfiguriert ist und der Berichtsanhang zum Anzeigen eines Analyseberichts jedes Parameters konfiguriert ist, der die Wellenformdaten und die numerischen Daten umfasst;
der Rahmen für den Bericht zur Multiparameteranalyse umfasst mindestens einen Bereich (201) mit grundlegenden Patienteninformationen;
und weiterhin umfasst:
einen oder mehrere der folgenden Parameterdaten-Trendanalysebereiche: einen Herzfrequenzdaten-Trendanalysebereich (202), einen Blutdruckdaten-Trendanalysebereich (203), einen Atemfrequenzdaten-Trendanalysebereich (204), einen Blutsauerstoffsättigungsgrad-Trendanalysebereich (205), einen Körpertemperaturdaten-Trendanalysebereich (206) und einen ausgewiesenen Datentrendanalysebereich (207); und
ein Bereich (208) für Schlussfolgerungen zur Datenanalyse;
die Anweisung zur Berichterstellung ferner einen Einstellungsparameter für den angegebenen Datentrendanalysebereich umfasst, gemäß dem der Anwendungsserver den Inhalt des angegebenen Datentrendanalysebereichs einstellt;
die Anweisung zur Berichterstellung außerdem einen Einstellungsparameter für den Datenanalyse-Abschlussbereich umfasst, gemäß dem der Anwendungsserver den Inhalt des Datenanalyse-Abschlussbereichs einstellt;
jeder der Parameterdaten-Trendanalysebereiche eine Ordinate als numerische Skala und eine Abszisse als Zeitskala umfasst, die zum Anzeigen eines Zustands in einer Zeiteinheit und eines Änderungsprozesses jedes Parameters konfiguriert ist und mindestens die statistischen Messwerte, die Zeitreihenkennungen und die Ereigniskennungen jedes Parameters umfasst;
der bezeichnete Datentrendanalysebereich zum Anzeigen eines Trendanalysediagramms von Parameterdaten konfiguriert ist, die voreingestellt sind und für Patienten mit unterschiedlichen Krankheiten eine intensive Überwachung erfordern; wobei die Parameterdaten, die eine intensive Überwachung erfordern, einen oder mehrere der folgenden Parameter umfassen: einen Perfusionsindex, einen Partialdruck von endtidalem Kohlendioxid, einen Atemwegsfluss/-druck, einen zentralvenösen Druck, einen Pulsdruck, ein Schlagvolumen, einen intrakraniellen Druck und einen Bispektralindex;
jeder der Parameterdatentrendanalysebereiche im Multiparameter-Analysebericht jeder Parameteranalyseseite im Berichtsanhang zugeordnet ist, und beim Klicken auf einen bestimmten Datentrendanalysebereich auf einem Bildschirm, auf dem der Multiparameter-Analysebericht angezeigt wird, der Multiparameter-Analysebericht automatisch zu einer entsprechenden Parameteranalyseseite springt, um einem Benutzer das Anzeigen der statistischen Messwerte, der statistischen Analysediagramme, der Wellenformdaten, der numerischen Daten und der markierten Ereignisdaten seitenweise zu ermöglichen und zum Anzeigen eine Vergrößerungs-, Verkleinerungs- oder Bildlaufoperation auf dem Bildschirm durchzuführen und einen Datenbereich zum Anzeigen auszuwählen.

9. Das System zum Generieren eines Datenanalyseberichts eines Multiparameter-Überwachungsgeräts nach Anspruch 7, wobei der Client mit der Berechtigung des ersten Modus und der Client mit der Berechtigung des zweiten Modus ferner dazu konfiguriert sind, den Multiparameter-Analysebericht, den Berichtsanhang und den eindeutigen Identifikationsindex an ein zugehöriges medizinisches Personalterminal (507) zu übertragen, nachdem der Multiparameter-Analysebericht und der Berichtsanhang generiert wurden;
das System ferner ein Terminal für medizinisches Personal umfasst, das so konfiguriert ist, dass es eine Anweisung zum Abrufen eines Berichts an den Anwendungsserver überträgt, wobei die Anweisung zum Abrufen des Berichts einen eindeutigen Identifikationsindex eines Patienten oder einen statistischen Parameter umfasst.
Der Anwendungsserver ist ferner dazu konfiguriert, die vom Terminal für medizinisches Personal übermittelte Anweisung zum Abrufen des Berichts zu empfangen, falls der eindeutige Kennungsindex enthalten ist, einen Multiparameter-Analysebericht und einen Berichtsanhang entsprechend dem eindeutigen Kennungsindex abzurufen und den Multiparameter-Analysebericht und den Berichtsanhang an das Terminal für medizinisches Personal zu übermitteln.
Falls der statistische Parameter enthalten ist, rufen Sie einen Multiparameter-Analysebericht und einen Berichtsanhang ab, die den statistischen Parameter erfüllen, und übertragen Sie den Multiparameter-Analysebericht und den Berichtsanhang an das Medizinisches Personalterminal.

10. Das System zum Generieren eines Datenanalyseberichts eines Multiparameter-Überwachungsgeräts nach Anspruch 9, wobei der Anwendungsserver außerdem dazu konfiguriert ist, eine von einem Client oder einem medizinischen Personalterminal mit einer Berichtsarchivierungsberechtigung übertragene Berichtsarchivierungsanweisung zu empfangen, wobei die Berichtsarchivierungsanweisung den eindeutigen Kennungsindex umfasst, einen Multiparameter-Analysebericht und einen Berichtsanhang entsprechend dem eindeutigen Kennungsindex abzurufen und den Multiparameter-Analysebericht und den Berichtsanhang an ein Krankenhausinformationsmanagementsystem, ein elektronisches Patientendatensystem oder ein kritisches klinisches Informationssystem zu übertragen, um den Multiparameter-Analysebericht und den Berichtsanhang in einer elektronischen Patientenakte zu speichern.

11. Das System zum Generieren eines Datenanalyseberichts eines Multiparameter-Überwachungsgeräts gemäß Anspruch 7, wobei das System außerdem einen Webserver (504) umfasst; und der Anwendungsserver so konfiguriert ist, dass er die Anweisung zur Berichtsgenerierung empfängt, die von einem Client-Browser übertragen und vom Webserver erfolgreich authentifiziert wurde, wobei die Anweisung zur Berichtsgenerierung eine Client-Browserkennung umfasst und die durch die Client-Browserkennung angegebene Berechtigung des Clients die Berechtigung des zweiten Modus ist;
das System außerdem ein mobiles Speichergerät umfasst; der Anwendungsserver so konfiguriert ist, dass er die vom Multiparameter-Überwachungsgerät übertragenen Multiparameterdaten empfängt, und umfasst:
das mobile Speichergerät (607) mit einer Schnittstelle des Multiparameter-Überwachungsgeräts verbunden wird, um die Multiparameter-Daten herunterzuladen; und
der Client stellt eine Verbindung zu einer Schnittstelle des mobilen Speichergeräts her, um gelesene Multiparameterdaten auf den Anwendungsserver hochzuladen.

## Revendications

1. Procédé de génération d'un rapport d'analyse de données d'un dispositif de surveillance multiparamétrique, **caractérisé en ce que** le procédé comprend les étapes
de laisser un serveur d'applications (403, 502, 602) recevoir des données multiparamétriques transmises en temps réel par le dispositif de surveillance multiparamétriques (405, 505, 604) et mémoriser les données multiparamétriques reçues ci-dessus dans un serveur de base de données (404, 503, 603) ;
de laisser un serveur d'applications (403, 502, 602) recevoir une instruction de génération de rapport transmise par un client (401, 501, 601), dans laquelle un indicateur du client est inclus et utilisé pour indiquer une autorité du client ; et
de laisser le serveur d'applications transmettre les données multiparamétriques au client de sorte que le client traite les données multiparamétriques pour générer un rapport d'analyse multiparamétrique (200) et une pièce jointe au rapport, si l'autorité du client est une autorité de premier mode ; et de laisser le serveur d'applications traiter les données multiparamétriques pour générer un rapport d'analyse multiparamétrique (200) et une pièce jointe au rapport et transmettre le rapport d'analyse multiparamétrique (200) et la pièce jointe au rapport au client, si l'autorité du client est une autorité de deuxième mode ;
après l'étape de laisser un serveur d'applications (403, 502, 602) recevoir des données multiparamétriques transmises en temps réel par le dispositif de surveillance multiparamétriques (405, 505, 604), le procédé comprend en outre les étapes
d'assigner un indicateur unique aux données multiparamétriques ;
de mémoriser l'indicateur unique assigné ci-dessus dans une première zone de mémoire du serveur de base de données et établir un index d'indicateur unique sur la base de l'indicateur unique ;
de mémoriser les données multiparamétriques reçues ci-dessus, dans une deuxième zone de mémoire du serveur de base de données, et faire une indication selon l'indicateur unique des données multiparamétriques ;
de mémoriser le rapport d'analyse multiparamétrique (200) et la pièce jointe au rapport générés ci-dessus, dans une troisième zone de mémoire du serveur de base de données, et faire une indication selon l'indicateur unique des données multiparamétriques ; et
de faire une mappe mutuelle de l'index d'indicateur unique établi dans la première zone de mémoire à la deuxième zone de mémoire ainsi que la troisième zone de mémoire, afin de gérer uniformément des informations mémorisées dans la première zone de mémoire, la deuxième zone de mémoire et la troisième zone de mémoire associées par l'indicateur unique ;
après l'étape de laisser un serveur d'applications (403, 502, 602) recevoir des données multiparamétriques transmises en temps réel par le dispositif de surveillance multiparamétriques (405, 505, 604), le procédé comprend en outre les étapes
de laisser le serveur d'applications (403, 502, 602) compter des durées des données multiparamétriques reçues ci-dessus ;
de transmettre l'index d'indicateur unique des données multiparamétriques les durées desquelles atteignent une durée prédéterminée au client ;
de transmettre l'index d'indicateur unique des données multiparamétriques les durées desquelles sont inférieures à une durée prédéterminée et n'augmentent plus au client ; et
de laisser le client émettre une instruction de génération de rapport au serveur d'applications en fonction de l'index d'indicateur unique reçu ci-dessus, dans laquelle des paramètres de période de données pour générer un rapport sont en outre inclus ;
le procédé comprend en outre les étapes
de laisser le serveur d'applications déterminer des données multiparamétriques correspondantes à une période qui doivent être traitées en fonction des paramètres de période de données pour générer un rapport, et de laisser le serveur d'applications générer un rapport d'analyse multiparamétrique et une pièce jointe au rapport en fonction des données multiparamétriques correspondantes à une période déterminées ci-dessus.

2. Procédé de génération d'un rapport d'analyse de données d'un dispositif de surveillance multiparamétrique selon la revendication 1, **caractérisé en ce que** les données multiparamétriques incluent des informations de patient, des données de forme d'onde et des données de type numérique ;
l'étape de générer un rapport d'analyse multiparamétrique et une pièce jointe au rapport comprend les sous-étapes
de calculer et traiter les données multiparamétriques pour obtenir des valeurs statistiques à mesure, des graphiques statistiques à analyse, des indicateurs chronologiques et des indicateurs d'événements, dans lesquels les valeurs statistiques à mesure incluent une valeur moyenne, une valeur maximale, une valeur minimale, une valeur d'analyse à variabilité ;
de mettre des informations de base de patient obtenues en faisant une mappe des valeurs statistiques à mesure, des indicateurs chronologiques, des indicateurs d'événements et de l'index d'indicateur unique dans un cadre de rapport d'analyse multiparamétrique, pour générer un rapport d'analyse multiparamétriques ; et
de mettre les valeurs statistiques à mesure, les graphiques statistiques à analyse, les indicateurs chronologiques, les indicateurs d'événements, des données de forme d'onde multiparamétriques et les données de type numérique dans un cadre d'une pièce jointe au rapport d'analyse multiparamétrique, pour générer une pièce jointe au rapport d'analyse multiparamétrique ;
le rapport d'analyse multiparamétriques est utilisé pour montrer une tendance de changement de chaque paramètre et une conclusion d'analyse de données, et la pièce jointe au rapport d'analyse multiparamétrique est utilisée pour montrer un rapport d'analyse de chaque paramètre, y compris les données de forme d'onde multiparamétriques et les données de type numérique;
le cadre de rapport d'analyse multiparamétrique comprend au moins une zone d'information de base de patient (201),
le cadre de rapport d'analyse multiparamétrique comprend en outre une ou plusieurs des zones d'analyse de tendance de données paramétriques suivantes :
une zone d'analyse de tendance de données de fréquence cardiaque (202), une zone d'analyse de tendance de données de pression artérielle (203), une zone d'analyse de tendance de données de fréquence respiratoire (204), une zone d'analyse de tendance de données de saturation en oxygène du sang (205), une zone d'analyse de tendance de données de température corporelle (206), et une zone d'analyse de tendance de données désignées (207) ;
ainsi qu'une zone de conclusion à analyse de données (208) ;
l'instruction de génération de rapport comprend en outre des paramètres de configuration qui indiquent la zone d'analyse de tendance de données, et le serveur d'applications fait assigner un contenu de la zone d'analyse de tendance de données indiquée ci-dessus en fonction des paramètres de configuration qui indiquent la zone d'analyse de tendance de données ;
l'instruction de génération de rapport comprend en outre des paramètres de configuration de la zone de conclusion à analyse de données, et le serveur d'applications fait assigner un contenu de la zone de conclusion à analyse de données en fonction des paramètres de configuration de la zone de conclusion à analyse de données ;
la zone d'analyse de tendance de données paramétriques est composée d'une ordonnée et d'une abscisse respectivement, dans lesquelles l'ordonnée est définie avec échelles numériques, et l'abscisse est définie avec échelles de temps, de manière à montrer des états, des changements et des processus d'évolution de divers paramètres en unité de temps, en outre la zone d'analyse de tendance de données paramétrique inclut au moins les valeurs statistiques à mesure, les indicateurs chronologiques et les indicateurs d'événements de chaque type des paramètres, la zone d'analyse de tendance de données indiquée est utilisée pour montrer un tableau d'analyse de tendance des données paramétriques prédéfinies qui doit être surveillées préférentiellement pour des patients de différentes maladies ;
les données paramétriques qui doit être surveillées préférentiellement sont un ou plusieurs des éléments suivants : un indice de perfusion, une pression partielle de dioxyde de carbone en fin d'expiration, un débit/pression de voies respiratoires, une pression veineuse centrale, une pression pulsée, un volume systolique, une pression intracrânienne, une indice bi-spectral de EEG ;
chaque zone d'analyse de tendance de données paramétriques du rapport d'analyse multiparamétrique est associée à chaque page d'analyse paramétrique dans la pièce jointe au rapport, de manière à cliquer une zone d'analyse de tendance de données sur l'écran qui montre le rapport d'analyse multiparamétrique pour se tourner automatiquement vers une page d'analyse paramétrique correspondante, feuilleter page par page les valeurs statistiques à mesure, les graphiques statistiques à analyse, les données de forme d'onde et les données de type numérique, ainsi que des données d'événement annotées, faire une amplification, une contraction, et une lecture en défilement sur l'écran, ainsi que sélectionner une section de données à feuilleter.

3. Procédé de génération d'un rapport d'analyse de données d'un dispositif de surveillance multiparamétrique selon la revendication 1, **caractérisé en ce que**
le client de l'autorité de premier mode et le client de l'autorité de deuxième mode également transmettent le rapport d'analyse multiparamétrique, la pièce jointe au rapport, et l'index d'indicateur unique à un terminal de médecin et infirmier associé (507), après la génération du rapport d'analyse multiparamétrique et de la pièce jointe au rapport ; le procédé comprend en outre les étapes
de laisser le serveur d'applications recevoir également une instruction d'acquisition de rapport transmise par le terminal de médecin et infirmier, dans laquelle un index d'indicateur unique d'un patient ou un paramètre statistique est inclus ;
de laisser le serveur d'applications extraire le rapport d'analyse multiparamétrique et la pièce jointe au rapport en fonction de l'index d'indicateur unique et les transmettre au terminal de médecin et infirmier, si l'index d'indicateur unique est inclus dans l'instruction d'acquisition de rapport ; et
de laisser le serveur d'applications extraire le rapport d'analyse multiparamétrique et la pièce jointe au rapport qui répondent au paramètre statistique et les transmettre au terminal de médecin et infirmier, si le paramètre statistique est inclus dans l'instruction d'acquisition de rapport.

4. Procédé de génération d'un rapport d'analyse de données d'un dispositif de surveillance multiparamétrique selon la revendication 3, **caractérisé en ce que** le procédé comprend en outre les étapes
de laisser le serveur d'applications recevoir également une instruction d'archivage de rapport transmise par un client qui dispose une autorité d'archivage de rapport ou par le terminal médecin et infirmier, dans laquelle l'index d'indicateur unique est inclus, et de laisser le serveur d'applications extraire le rapport d'analyse multiparamétrique (200) et la pièce jointe au rapport en fonction de l'index d'indicateur unique, et les transmettre à un système de gestion d'information de l'hôpital, à un système de dossier électronique ou à un système d'information clinique d'ICU, et les mémoriser dans un dossier électronique du patient.

5. Procédé de génération d'un rapport d'analyse de données d'un dispositif de surveillance multiparamétrique selon la revendication 1, **caractérisé en ce que** l'étape de laisser un serveur d'applications (403, 502, 602) recevoir une instruction de génération de rapport transmise par un client (401, 501, 601) comprend en outre les sous-étapes
de laisser le serveur d'applications recevoir une instruction de génération de rapport transmise par un navigateur d'client authentifié par un serveur de WEB (504), dans laquelle un indicateur de navigateur de client est inclus, et
de nommer l'autorité du client indiqué par l'indicateur de navigateur de client à l'autorité de deuxième mode.

6. Procédé de génération d'un rapport d'analyse de données d'un dispositif de surveillance multiparamétrique selon la revendication 1, **caractérisé en ce que** l'étape de laisser un serveur d'applications (403, 502, 602) recevoir des données multiparamétriques transmises en temps réel par le dispositif de surveillance multiparamétriques (405, 505, 604) comprend les sous-étapes
de laisser un dispositif de mémoire amovible (607) connecter l'interface du dispositif de surveillance multiparamétriques pour télécharger des données multiparamétriques ; et
de laisser le client connecter l'interface du dispositif de surveillance multiparamétriques pour mettre en ligne les données multiparamétriques téléchargées au serveur d'applications.

7. Système de génération d'un rapport d'analyse de données d'un dispositif de surveillance multiparamétrique, **caractérisé en ce que** le système comprend
un ou plusieurs clients (401, 501, 601) qui transmettent une instruction de génération de rapport à un serveur d'applications (403, 502, 602), dans laquelle un indicateur du client est inclus et utilisé pour indiquer une autorité du client ;
le serveur d'applications (403, 502, 602) étant configuré pour recevoir des données multiparamétriques transmises en temps réel par un dispositif de surveillance multiparamétriques (405, 505, 604) et pour recevoir une instruction de génération de rapport transmise par un client (401, 501, 601), de manière à laisser le serveur d'applications transmettre les données multiparamétriques au client de sorte que le client traite les données multiparamétriques pour générer un rapport d'analyse multiparamétrique (200) et une pièce jointe au rapport, si l'autorité du client est une autorité de premier mode ; et de manière à laisser le serveur d'applications traiter les données multiparamétriques pour un rapport d'analyse multiparamétrique (200) et une pièce jointe au rapport et transmettre le rapport d'analyse multiparamétrique (200) et la pièce jointe au rapport au client, si l'autorité du client est une autorité de deuxième mode ;
un serveur de base de données (404, 503, 603) configuré pour mémoriser les données multiparamétriques reçues par le serveur d'applications ;
le serveur d'applications (403, 502, 602) étant en outre configuré pour assigner un indicateur unique aux données multiparamétriques après recevoir les données multiparamétriques transmises en temps réel par le dispositif de surveillance multiparamétriques (405, 505, 604) ;
le serveur de base de données (404, 503, 603) ayant une première zone de mémoire, une deuxième zone de mémoire et une troisième zone de mémoire, dans lesquelles
la première zone de mémoire du serveur de base de données est configurée pour mémoriser l'indicateur unique assigné ci-dessus et pour établir un index d'indicateur unique sur la base de l'indicateur unique ;
la deuxième zone de mémoire du serveur de base de données est configurée pour mémoriser les données multiparamétriques reçues par le serveur d'applications, et pour faire une indication selon l'indicateur unique des données multiparamétriques ; et
la troisième zone de mémoire du serveur de base de données est configurée pour mémoriser le rapport d'analyse multiparamétrique (200) et la pièce jointe au rapport générés, et pur faire une indication selon l'indicateur unique des données multiparamétriques ;
de manière à faire une mappe mutuelle de l'index d'indicateur unique établi dans la première zone de mémoire à la deuxième zone de mémoire ainsi que la troisième zone de mémoire, afin de gérer uniformément des informations mémorisées dans la première zone de mémoire, la deuxième zone de mémoire et la troisième zone de mémoire associées par l'indicateur unique ;
le serveur d'applications (403, 502, 602) étant en outre configuré pour compter des durées des données multiparamétriques reçues après recevoir des données multiparamétriques transmises en temps réel par le dispositif de surveillance multiparamétriques (405, 505, 604),
de manière à transmettre l'index d'indicateur unique des données multiparamétriques les durées desquelles atteignent une durée prédéterminée au client, et à transmettre l'index d'indicateur unique des données multiparamétriques les durées desquelles sont inférieures à une durée prédéterminée et n'augmentent plus au client ;
le client étant en outre configuré pour émettre une instruction de génération de rapport au serveur d'applications en fonction de l'index d'indicateur unique reçu, dans laquelle des paramètres de période de données pour générer un rapport sont en outre inclus ;
le serveur d'applications étant en outre configuré pour déterminer des données multiparamétriques correspondantes à une période qui doivent être traitées en fonction des paramètres de période de données pour générer un rapport, et pour générer un rapport d'analyse multiparamétrique et une pièce jointe au rapport en fonction des données multiparamétriques correspondantes à une période déterminées ci-dessus.

8. Système de génération d'un rapport d'analyse de données d'un dispositif de surveillance multiparamétrique selon la revendication 7, **caractérisé en ce que**
les données multiparamétriques incluent des informations de patient, des données de forme d'onde et des données de type numérique ;
le système exécute une étape de générer un rapport d'analyse multiparamétrique et une pièce jointe au rapport qui comprend les sous-étapes
de calculer et traiter les données multiparamétriques pour obtenir des valeurs statistiques à mesure, des graphiques statistiques à analyse, des indicateurs chronologiques et des indicateurs d'événements, dans lesquels les valeurs statistiques à mesure incluent une valeur moyenne, une valeur maximale, une valeur minimale, une valeur d'analyse à variabilité ;
de mettre des informations de base de patient obtenues en faisant une mappe des valeurs statistiques à mesure, des indicateurs chronologiques, des indicateurs d'événements et de l'index d'indicateur unique dans un cadre de rapport d'analyse multiparamétrique, pour générer un rapport d'analyse multiparamétriques ; et
de mettre les valeurs statistiques à mesure, les graphiques statistiques à analyse, les indicateurs chronologiques, les indicateurs d'événements, des données de forme d'onde multiparamétriques et les données de type numérique dans un cadre d'une pièce jointe au rapport d'analyse multiparamétrique, pour générer une pièce jointe au rapport d'analyse multiparamétrique ;
le rapport d'analyse multiparamétriques est utilisé pour montrer une tendance de changement de chaque paramètre et une conclusion d'analyse de données, et la pièce jointe au rapport d'analyse multiparamétrique est utilisée pour montrer un rapport d'analyse de chaque paramètre, y compris les données de forme d'onde multiparamétriques et les données de type numérique;
le cadre de rapport d'analyse multiparamétrique comprend au moins une zone d'information de base de patient (201),
le cadre de rapport d'analyse multiparamétrique comprend en outre une ou plusieurs des zones d'analyse de tendance de données paramétriques suivantes :
une zone d'analyse de tendance de données de fréquence cardiaque (202), une zone d'analyse de tendance de données de pression artérielle (203), une zone d'analyse de tendance de données de fréquence respiratoire (204), une zone d'analyse de tendance de données de saturation en oxygène du sang (205), une zone d'analyse de tendance de données de température corporelle (206), et une zone d'analyse de tendance de données désignées (207) ;
ainsi qu'une zone de conclusion à analyse de données (208) ;
l'instruction de génération de rapport comprend en outre des paramètres de configuration qui indiquent la zone d'analyse de tendance de données, et le serveur d'applications fait assigner un contenu de la zone d'analyse de tendance de données indiquée ci-dessus en fonction des paramètres de configuration qui indiquent la zone d'analyse de tendance de données ;
l'instruction de génération de rapport comprend en outre des paramètres de configuration de la zone de conclusion à analyse de données, et le serveur d'applications fait assigner un contenu de la zone de conclusion à analyse de donnée en fonction des paramètres de configuration de la zone de conclusion à analyse de données ;
la zone d'analyse de tendance de données paramétriques est composée d'une ordonnée et d'une abscisse respectivement, dans lesquelles l'ordonnée est définie avec échelles numériques, et l'abscisse est définie avec échelles de temps, de manière à montrer des états, des changements et des processus d'évolution de divers paramètres en unité de temps, en outre la zone d'analyse de tendance de données paramétrique inclut au moins les valeurs statistiques à mesure, les indicateurs chronologiques et les indicateurs d'événements de chaque type des paramètres ;
la zone d'analyse de tendance de données indiquée est utilisée pour montrer un tableau d'analyse de tendance des données paramétriques prédéfinies qui doit être surveillées préférentiellement pour des patients de différentes maladies ;
les données paramétriques qui doit être surveillées préférentiellement sont un ou plusieurs des éléments suivants : un indice de perfusion, une pression partielle de dioxyde de carbone en fin d'expiration, un débit/pression de voies respiratoires, une pression veineuse centrale, une pression pulsée, un volume systolique, une pression intracrânienne, une indice bi-spectral de EEG ;
chaque zone d'analyse de tendance de données paramétriques du rapport d'analyse multiparamétrique est associée à chaque page d'analyse paramétrique dans la pièce jointe au rapport, de manière à cliquer une zone d'analyse de tendance de données sur l'écran qui montre le rapport d'analyse multiparamétrique pour se tourner automatiquement vers une page d'analyse paramétrique correspondante, feuilleter page par page les valeurs statistiques à mesure, les graphiques statistiques à analyse, les données de forme d'onde et les données de type numérique, ainsi que des données d'événement annotées, faire une amplification, une contraction, et une lecture en défilement sur l'écran, ainsi que sélectionner une section de données à feuilleter.

9. Système de génération d'un rapport d'analyse de données d'un dispositif de surveillance multiparamétrique selon la revendication 7, **caractérisé en ce que**
le client de l'autorité de premier mode et le client de l'autorité de deuxième mode également transmettent le rapport d'analyse multiparamétrique, la pièce jointe au rapport, et l'index d'indicateur unique à un terminal de médecin et infirmier associé (507), après la génération du rapport d'analyse multiparamétrique et de la pièce jointe au rapport ;
le système comprend en outre un terminal de médecin et infirmier qui est configuré pour transmettre une instruction d'acquisition de rapport au serveur d'applications recevoir, dans laquelle un index d'indicateur unique d'un patient ou un paramètre statistique est inclus ;
le serveur d'applications est également configuré pour recevoir une instruction d'acquisition de rapport transmis par le terminal de médecin et infirmier,
afin que le serveur d'applications extraie le rapport d'analyse multiparamétrique et la pièce jointe au rapport en fonction de l'index d'indicateur unique et les transmette au terminal de médecin et infirmier, si l'index d'indicateur unique est inclus dans l'instruction d'acquisition de rapport ; et
afin que le serveur d'applications extraie le rapport d'analyse multiparamétrique et la pièce jointe au rapport qui répondent au paramètre statistique et les transmette au terminal de médecin et infirmier, si le paramètre statistique est inclus dans l'instruction d'acquisition de rapport.

10. Système de génération d'un rapport d'analyse de données d'un dispositif de surveillance multiparamétrique selon la revendication 9, **caractérisé en ce que**
le serveur d'applications est également configuré pour recevoir une instruction d'archivage de rapport transmise par un client qui dispose une autorité d'archivage de rapport ou par le terminal médecin et infirmier, dans laquelle l'index d'indicateur unique est inclus, et le serveur d'applications extrait le rapport d'analyse multiparamétrique et la pièce jointe au rapport en fonction de l'index d'indicateur unique, et les transmet à un système de gestion d'information de l'hôpital, à un système de dossier électronique ou à un système d'information clinique d'ICU, et les mémorise dans un dossier électronique du patient.

11. Système de génération d'un rapport d'analyse de données d'un dispositif de surveillance multiparamétrique selon la revendication 7, **caractérisé en ce que**
le système comprend en outre un serveur de WEB (504),
le serveur d'applications reçoit une instruction de génération de rapport transmise par un navigateur d'client authentifié par le serveur de WEB (504), dans laquelle un indicateur de navigateur de client est inclus, et l'autorité du client indiqué par l'indicateur de navigateur de client est nommée à l'autorité de deuxième mode.
le système comprend en outre un dispositif de mémoire amovible,
le serveur d'applications est configuré pour recevoir des données multiparamétriques transmises en temps réel par le dispositif de surveillance multiparamétriques de manière à laisser le dispositif de mémoire amovible (607) connecter l'interface du dispositif de surveillance multiparamétriques pour télécharger des données multiparamétriques ; et
de manière à laisser le client connecter l'interface du dispositif de surveillance multiparamétriques pour mettre en ligne les données multiparamétriques téléchargées au serveur d'applications.
